(19)

**Europäisches Patentamt**

**European Patent Office**

**Office européen des brevets**

(11) **EP 4 574 109 A1**

(12) **EUROPÄISCHE PATENTANMELDUNG**

(43) Veröffentlichungstag:
   **25.06.2025 Patentblatt 2025/26**

(21) Anmeldenummer: **23219548.7**

(22) Anmeldetag: **21.12.2023**

(51) Internationale Patentklassifikation (IPC):
   ***A61F 9/008*** *(2006.01)*

(52) Gemeinsame Patentklassifikation (CPC):
   **A61F 9/00821;** A61F 2009/00844;
   A61F 2009/00863

(84) Benannte Vertragsstaaten:
   **AL AT BE BG CH CY CZ DE DK EE ES FI FR GB
   GR HR HU IE IS IT LI LT LU LV MC ME MK MT NL
   NO PL PT RO RS SE SI SK SM TR**
   Benannte Erstreckungsstaaten:
   **BA**
   Benannte Validierungsstaaten:
   **KH MA MD TN**

(71) Anmelder: **OD-OS MacuTherm GmbH
   14513 Teltow (DE)**

(72) Erfinder:
   • **Rose, Arnd
     14532 Stahnsdorf (DE)**
   • **Paulauskas, Gediminas
     12165 Berlin (DE)**
   • **Zogkas, Alexandros
     10779 Berlin (DE)**

(74) Vertreter: **Pfenning, Meinig & Partner mbB
   Patent- und Rechtsanwälte
   Joachimsthaler Straße 10-12
   10719 Berlin (DE)**

(54) **PROZESSSYSTEM ZUR BEHANDLUNG DER NETZHAUT EINES AUGES**

(57) Die Erfindung bezieht sich auf ein Prozesssystem (200) zur Behandlung der Netzhaut (16) eines Auges (4) mittels einer Behandlungslichtquelle (2), insbesondere eines Behandlungslasers,
-mit einem Datenerfassungs-und Behandlungssystem (202), das eine Datenerfassungseinrichtung (202a) zur Erfassung wenigstens einer Aufnahme der Netzhaut, insbesondere durch eine mit einem Sensor ausgestattete Aufnahmeeinrichtung oder durch Übernahme von gespeicherten Messdaten, und eine Behandlungseinrichtung (202b) zur Behandlung der Netzhaut mit der Behandlungslichtquelle in Abhängigkeit von der wenigstens einen Aufnahme der Netzhaut umfasst, und
-mit einer Prozesssteuerungseinrichtung (201), die mit dem Datenerfassungs-und Behandlungssystem, insbesondere mit der Datenerfassungseinrichtung einerseits und mit der Behandlungseinrichtung andererseits verbunden ist und die dazu eingerichtet ist, in Abhängigkeit von den durch die Datenerfassungseinrichtung erfassten Daten Steuerungsdaten zur Steuerung der Behandlungseinrichtung zu erzeugen, wobei das Prozesssystem dazu eingerichtet ist, die Datenerfassung, die Erzeugung von Steuerungsdaten sowie die Behandlung automatisiert ablaufen zu lassen, und wobei das Prozesssystem entweder dazu eingerichtet ist, vor Beginn der Behandlung mittels der Behandlungseinrichtung den weiteren Verlauf des Verfahrens bis zu einer Freigabe (206) zu unterbrechen und nach einer Freigabe fortzusetzen, oder dazu eingerichtet ist, dass durch die Prozesssteuerungseinrichtung entschieden wird, ob das Verfahren einschließlich der Behandlung automatisiert ohne eine Unterbrechung durchgeführt oder vor Beginn der Behandlung unterbrochen und nur nach einer Freigabe automatisiert fortgesetzt wird.

EP 4 574 109 A1

**Beschreibung**

Die Erfindung liegt auf dem Gebiet der Medizintechnik, insbesondere der Augenheilkunde und ist mit besonderen Vorteilen bei Laser- Augenbehandlungen einsetzbar, beispielsweise bei einer Behandlung einer altersbedingten Makula-Degeneration, jedoch grundsätzlich für die Behandlung aller Zustände der Netzhaut eines Auges, die sich mit einer Licht- und/oder Wärmeeinwirkung beeinflussen lassen.

Augenbehandlungen mittels einer Lichtquelle, insbesondere eines Lasers, sind für viele Krankheiten und Alterungszustände und zur Verfolgung verschiedener Ziele üblich. Oft erzeugen derartige Behandlungen eine Gewebeveränderung. Die Wirkung beruht dabei weitgehend auf dem kontrollierten Eintrag von Energie in Form von Lichtenergie in die Netzhaut oder netzhautnahe Bereiche, um dort beispielsweise durch eine Temperaturerhöhung die Veränderung, beispielsweise Koagulation, von Zellen zu erreichen. Die Laserleistung kann bei manchen Anwendungen auch so hoch gewählt werden, dass Zellen gezielt thermisch zerstört werden.

Wegen der bestehenden Risiken und der komplexen Aufgabenstellungen werden Laserbehandlungen am Auge eines Patienten auf der Grundlage von Vermessungen der Netzhaut dezidiert geplant, um die Schädigung von zu behandelnden und von nicht zu behandelnden Geweberegionen zu verhindern oder zu minimieren. Derartige Behandlungen werden aus diesem Grund auch weitestgehend durch Ärzte oder unter enger Kontrolle von Ärzten durchgeführt. Insbesondere muss die Laserleistung eines Behandlungslasers gut kontrolliert und auf die individuellen Bedingungen des Patienten abgestimmt werden, um bestimmte Gewebeeinflüsse zu erreichen und dabei ungewollte Gewebeschädigungen zu vermeiden. Für diesen Zweck werden verschiedene Kontroll- und Untersuchungsmethoden genutzt, um die physiologischen Bedingungen im Auge der Patienten vor einer Behandlung zu ermitteln und bei der Behandlungsplanung zu berücksichtigen.

Es ist derzeit üblich, die individuellen Behandlungsbedingungen im Auge eines Patienten durch sogenannte Titrationsschüsse mit dem Behandlungslaser zu testen, wobei eine sichtbare Gewebeveränderung nach den Titrationsschüssen kontrolliert wird. Die sichtbaren Ergebnisse werden dann für die Kalibrierung eines Behandlungslasers genutzt.

Oft wird vor einer Netzhautbehandlung zunächst eine Abbildung der Netzhaut des potenziell zu behandelnden Auges erfasst und aufgrund von Gegebenheiten, die mittels der Abbildung erkennbar sind, über die Notwendigkeit und/oder Art und Intensität einer Behandlung entschieden.

Aus der europäischen Patentanmeldung EP 1 875 857 A1 ist ein Ophthalmoskop mit einer Abbildungsoptik und einer Beleuchtungseinrichtung sowie einem optischen Sensor bekannt, mit dem Bilder der Netzhaut eines Auges aufgenommen werden können. Ein solches Ophthalmoskop kann beispielsweise für die Planung einer Laserbehandlung durch einen Operateur genutzt werden.

Der vorliegenden Erfindung liegt vor dem Hintergrund des Standes der Technik die Aufgabe zugrunde, die Durchführung von Netzhautbehandlungen zu vereinfachen.

Die Aufgabe wird durch die Erfindung mittels eines Prozesssystems sowie mittels eines Datenerfassungs-und Behandlungssystems zur Behandlung der Netzhaut eines Auges und mittels eines Verfahrens zur Behandlung der Netzhaut eines Auges entsprechend den unabhängigen Patentansprüchen gelöst. Die abhängigen Patentansprüche stellen mögliche Implementierungen der Erfindung vor.

Demgemäß bezieht sich die Erfindung auf ein Prozesssystem zur Behandlung der Netzhaut eines Auges mittels einer Behandlungslichtquelle, insbesondere eines Behandlungslasers,

- mit einem Datenerfassungs-und Behandlungssystem, das eine Datenerfassungseinrichtung zur Erfassung wenigstens einer Aufnahme der Netzhaut, insbesondere durch eine mit einem Sensor ausgestattete Aufnahmeeinrichtung oder durch Übernahme von bereits existierenden Messdaten, und eine Behandlungseinrichtung zur Behandlung der Netzhaut mit der Behandlungslichtquelle in Abhängigkeit von der wenigstens einen Aufnahme der Netzhaut umfasst, und

- mit einer Prozesssteuerungseinrichtung, die mit dem Datenerfassungs-und Behandlungssystem, insbesondere mit der Datenerfassungseinrichtung einerseits und mit der Behandlungseinrichtung andererseits, verbunden ist und die dazu eingerichtet ist, in Abhängigkeit von den durch die Datenerfassungseinrichtung erfassten Daten Steuerungsdaten zur Steuerung der Behandlungseinrichtung zu erzeugen. Zur Lösung der Aufgabe ist dabei vorgesehen, dass das Prozesssystem dazu eingerichtet ist, die Datenerfassung, die Erzeugung von Steuerungsdaten sowie die Behandlung automatisiert ablaufen zu lassen, wobei das Prozesssystem entweder dazu eingerichtet ist, vor Beginn der Behandlung mittels der Behandlungseinrichtung den weiteren Verlauf des Verfahrens bis zu einer Freigabe zu unterbrechen und nach einer Freigabe fortzusetzen, oder dazu eingerichtet ist, dass durch die Prozesssteuerungseinrichtung entschieden wird, ob das Verfahren einschließlich der Behandlung automatisiert ohne eine Unterbrechung durchgeführt oder vor Beginn der Behandlung unterbrochen und nur nach einer Freigabe automatisiert fortgesetzt wird.

In der Vergangenheit waren Augenbehandlungen und unter diesen besonders Netzhautbehandlungen durch einen hohen Aufwand bei der Vermessung des Auges und der Durchführung der Behandlung geprägt. Es sind bereits Verfahren bekannt geworden, die einzelne Verfahrensschritte wie beispielsweise die Behandlungsplanung, durch eine teilweise Automatisierung unterstützen sollen. Die vorliegende Erfindung stellt jedoch das erste System zur fallweise vollständigen oder weit überwiegenden Automatisierung eines umfassenden Gesamtprozesses vor.

Hierzu kann die Netzhaut eines Patienten zunächst in einer Datenerfassungseinrichtung vermessen werden. Eine solche Datenerfassungseinrichtung kann eine oder mehrere verschiedene Aufnahmevorrichtungen mit Sensoren zur Erzeugung von Aufnahmen der Netzhaut oder von Teilen der Netzhaut aufweisen. Solche Aufnahmevorrichtungen können Kameras, beispielsweise zur Aufnahme von Farb-Fundusfotografien oder schwarz/weiß-Kameras oder optische Scanner oder entsprechende Bildaufnahmegeräte zur Aufnahme von Bildern im Infrarot- oder UV- Bereich sowie Fluoreszenz- Bildaufnahmegeräte oder OCT- Geräte zur optischen Kohärenztomographie aufweisen.

Es kann auch beispielsweise vorgesehen sein, dass als bildgebende Verfahren in der Datenerfassungseinrichtung eines, zwei oder mehr der folgenden Verfahren verwendet werden:
Fotografische Farbbildaufnahme, fotografische Infrarotaufnahme (IR-Fotografie), OCT-B-scan (Tiefenschnittbild einer mit optischer Kohärenztomografie erstellten Aufnahme), BAF (Blue Autofluorescence, Fluoreszenzaufnahme ohne zusätzlichen Farbstoff, Anregungswellenlänge 488nm), Scanning laser ophtalmoscopy, OCT in Verbindung mit Scanning laser ophtalmoscopy), FAG (Fluorescence Angiography, Fluoreszenzaufnahme mit Farbstoff Fluorescein, Anregungswellenlänge 488nm).

Es kann somit beispielsweise vorgesehen sein, dass eine Netzhautaufnahme mittels Fundus-Farbfotografie mit einer IR-Fotografie kombiniert wird oder mit einer OCT- B-Aufnahme oder mit einer blue-Autofluoreszenzaufnahme (BAF) oder mit einer Scanning-Laser-Ophthalmoskopie- Aufnahme (SLO), oder es können mehrere Fundus-Farbfotografien bei verschiedenen Beleuchtungsintensitäten oder verschiedenen Beleuchtungsspektren oder aus verschiedenen Blickwinkeln oder mit verschiedenen Beleuchtungswinkeln miteinander kombiniert werden oder eine IR- Fotografie mit eine OCT- Aufnahme oder einer SLO. Es kann auch eine OCT -Aufnahme mit einer SLO oder eine von diesen mit BAF kombiniert werden. Jede der genannten Methoden kann auch beispielsweise mit einer FAG- Aufnahme kombiniert werden. Es können auch mehrere Fundus- Farbfotografien oder IR- Aufnahmen unter verschiedenen Aufnahmebedingungen miteinander kombiniert werden oder mehrere Fundus- Farbfotografien oder IR-Aufnahmen unter verschiedenen Aufnahmebedingungen mit einem anderen der genannten Verfahren.

Durch diesen Ansatz werden nicht nur mehr Daten zur Verfügung gestellt, sondern es werden auch die Korrelationen zwischen den verschiedenen Arten der Netzhautaufnahmen und die Redundanzen/Überlappungen des Informationsgehaltes ermittelt und für die Erzeugung von Steuerungsdaten durch das Prozesssystem zur Verfügung gestellt.

Die Datenerfassungseinrichtung kann Vermessungsdaten zu Aufnahmen der oben beschriebenen Arten auch direkt übernehmen, wenn derartige Aufnahmen bereits aus einer früheren Messung oder von einer anderen Datenerfassungseinrichtung aufgenommen existieren. Dazu können entsprechende Datensätze, die gespeichert vorliegen können, über eine digitale Kommunikationsschnittstelle an die Datenerfassungseinrichtung des Prozesssystems elektronisch übermittelt werden, beispielsweise mittels einer online- Verbindung, eines Downloads durch das Prozesssystem, oder durch eine elektronische mail. Solche Datensätze können auch durch ein mobliles Endgerät übersendet werden, entweder mittels einer üblichen Fernkommunikation oder auch durch eine Kommunikation mit begrenzter Reichweite, beispielsweise gemäß den Standards bluetooth, wifi oder über eine NFC- Schnittstelle oder auch leitungsgebunden, so dass ein Patient diese Datensätze , gespeichert in seinem Smartphone oder auf einem Speicherstick, zu dem Prozesssystem mitbringen kann. Zusätzlich zu der Datenerfassung durch Vermessung des Auges eines Patienten oder durch die Übernahme von Vermessungsdaten oder Aufnahmen des Auges/der Netzhaut von einem Dritten kann die Datenerfassungseinrichtung auch dazu dienen, wenigstens einen unabhängig von der Netzhaut des Patienten ermittelter Parameter, insbesondere einen Hautparameter, einen Haarparameter, einen Irisparameter, das Alter des Patienten, eine physiologisch wirksame Lebensgewohnheit oder eine Vorerkrankung zu erfassen. Die genannten Daten können entweder durch eine Messung oder durch eine Dateneigabe oder die Übermittlung von Daten aus einem anderen System in dem Prozesssystem, insbesondere in dem Datenerfassungs- und Behandlungssystem, weiter insbesondere in der Datenerfassungseinrichtung, erfasst und durch die Prozesssteuerungseinrichtung zur Erzeugung von Steuerungsdaten berücksichtigt werden. Beispielsweise können Hautparameter, Haarparameter und Irisparameter durch geeignete Sensoren wie Farb- oder Hyperspektralkameras, Reflektometer oder Irisscanner erfasst werden.

Eine Netzhautaufnahme für sich oder eine Gruppe von verschiedenen Netzhautaufnahmen mit teilweise überlappendem Informationsgehalt erlaubt bereits die Ableitung einer Vielzahl von Informationen, aus denen mittels eines Vorhersagemodells in der Prozesssteuerungseinrichtung bereits Bestrahlungsintensitätswerte für bestimmte Zwecke einer Netzhautbestrahlung ermittelt werden können.

Als Hautparameter, Haarparameter und Irisparameter kann dabei eine Pigmentierungsstärke oder-Dichte gemessen werden, weil diese in gewissem Umfang mit der Pigmentierungsstärke der Netzhaut korreliert. Die Pigmentierungsstärke kann dabei beispielsweise mittels einer wellenlängenabhängigen Reflektivitätsmessung ermittelt werden. Jedoch kann auch der Hautparameter ebenso wie der Haarparameter oder der Irisparameter durch eine Farbmessung bestimmt werden, indem beispielsweise eine fotografische Aufnahme, insbesondere eine Farbaufnahme oder IR-Aufnahme oder Live-Ansicht analysiert wird und die Farbe in einem Farbraum, beispielsweise im RGB- oder HSV-Farbraum, anhand der Intensitäten in den jeweiligen Farbkanälen analysiert wird.

Eine physiologisch wirksame Lebensgewohnheit des Patienten kann beispielsweise die Gewohnheit, zu rauchen, regelmäßiger Alkoholgenuss oder anderer Drogengebrauch oder Übergewicht sein, während als Vorerkrankung beispielsweise Diabetes oder hoher Blutdruck in Frage kommt. Die letztgenannten Daten sowie das Alter eines Patienten werden bevorzugt durch eine Dateneingabe oder die Übernahme von Daten aus anderen Systemen in das Prozesssystem übernommen.

Die optionale Zusammenstellung aus einem unabhängig von der Netzhaut des Patienten ermittelten Parameter des Patienten und wenigstens einer Netzhautaufnahme erlaubt eine objektive und dabei schnelle und einfache Datenerhebung. Die Nutzung einer Netzhautaufnahme für sich kann zudem erlauben, beispielsweise Verletzungen der Netzhaut oder Veränderungen im Vergleich zu einem früheren, bereits dokumentierten, Zustand zu detektieren und zu behandeln.

Unter Berücksichtigung der durch die Datenerfassungseinrichtung erfassten Daten kann die Prozesssteuerungseinrichtung Steuerungsdaten zur Steuerung der Behandlung der Netzhaut mit dem Behandlungssystem erzeugen. Dabei kann die Prozesssteuerungseinrichtung auch zusätzlich zu den durch die Datenerfassungseinrichtung erfassten Daten eine ganz oder teilweise durch einen Arzt erstellte Behandlungsplanung oder durch den Arzt eingegebene Vorgaben oder Korrekturen berücksichtigen. Es kann auch vorgesehen sein, dass die durch die Prozesssteuerungseinrichtung erzeugten Steuerungsdaten, beispielsweise eine erstellte Behandlungsplanung, durch einen Arzt ganz oder teilweise ergänzt oder korrigiert werden. Die Prozesssteuerungseinrichtung kann als Datenverarbeitungseinrichtung ausgebildet sein, die dazu eingerichtet ist, nach einer oder mehreren Regeln bestimmten aus erfassten Daten des Patienten bestimmte Behandlungsparameter, beispielsweise auch jeweils für bestimmte Oberflächenabschnitte der Netzhaut, zu ermitteln oder aus einer Mehrzahl von Behandlungsoptionen eine oder mehrere Optionen zur Behandlung der individuellen Netzhaut auszuwählen. Die Prozesssteuerungseinrichtung kann auch als eine selbstlernende Einrichtung, beispielsweise ein trainiertes neuronales Netz ausgebildet sein oder eine solche Einrichtung enthalten.

Beispielsweise kann eine durch die Prozesssteuerungseinrichtung auswählbare Behandlungsoption eine Behandlung mit einer Behandlungslichtquelle und einer Intensität oder Intensitätsverteilung sein, durch die die Netzhaut sicher nicht geschädigt wird. Es sind in diesem Zusammenhang Intensitätsschwellen bekannt, unterhalb deren eine bleibende Netzhautschädigung oder -veränderung ausgeschlossen werden kann. Dennoch kann die Behandlung mit einer Intensität unterhalb einer gewebeverändernden Schwelle physiologische Vorgänge in der Netzhaut oder im umliegenden Gewebe initiieren, unterstützen oder beschleunigen. Kann die Prozesssteuerungseinrichtung eine solche Behandlungsoption als sinnvoll auswählen, so kann sie beispielsweise entscheiden, dass die Behandlung ohne die Notwendigkeit einer Freigabe automatisiert bis zum Abschluss durchgeführt werden kann. Dies kann auch für eine nur teilweise Behandlung der Netzhaut, also nur in bestimmten Flächenbereichen oder Abschnitten, gelten. Die Analyse, ob und /oder in welchen Flächenbereichen eine Netzhaut ohne ein weiteres Anhalten des Prozesses durchgeführt werden kann, kann durch die Prozesssteuerungseinrichtung auf der Basis der Netzhautaufnahmen getroffen werden. Die Erfindung bezieht sich jedoch auch auf ein Prozesssystem, welches grundsätzlich ein Anhalten/eine Prozessunterbrechung vorsieht und bei dem unbedingt eine Freigabe erfolgen muss, bevor das Verfahren mit einer Behandlung durch die Bestrahlung fortgesetzt wird. Zu diesem Zweck kann das Prozesssystem eine Freigabeaufforderung erzeugen und verschicken oder für einen Adressaten sichtbar machen, wobei der Adressat zur Überprüfung wenigstens eines Teils der vorgeschlagenen Steuerungsdaten aufgefordert wird und ihm die Möglichkeit gegeben wird, die Steuerungsdaten vor der Freigabe zu ergänzen und/oder zu korrigieren. Unter der Freigabe kann somit außer der reinen Akzeptanz eines Vorschlages der Prozesssteuerungseinrichtung auch die vorgelagerte Ergänzung, Ersetzung und Korrektur der Steuerungsdaten durch die freigebende Person oder Instanz einschließlich der finalen Akzeptanz verstanden werden. Die Steuerungsdaten können in diesem Zusammenhang sowohl eine konkrete Behandlungsplanung als auch weitere Parameter bis zu den Daten zur technischen Laseransteuerung bei der Behandlung umfassen. Beispielsweise kann für eine freigebende Instanz besonders der Behandlungsplan für eine Überprüfung und gegebenenfalls Korrektur oder Ergänzung in Frage kommen.

Eine Freigabesignal kann, sowohl im Falle der unbedingten Notwendigkeit als auch im Falle der Festlegung durch die Prozesssteuerungseinrichtung, dass eine Prozessunterbrechung erfolgt und der Prozess nur nach einer Freigabe fortgesetzt wird, entweder durch ein Freigabemodul in dem Prozesssystem oder durch weiteres automatisiertes, von dem Prozesssystem unabhängiges System oder durch eine Bedienperson, insbesondere einen Arzt, übermittelt werden. Zu dem Zweck, die Entscheidungsqualität zu sichern, kann das Prozesssystem ein Identifizierungsmodul aufweisen, in dem eine zugelassene Person oder ein System sich identifiziert und/oder eine Autorisierung nachweist. Verfahren für eine derartige Identifizierung/Authentifizierung sind an sich bekannt und können durch softwaregestützte Zertifikate oder Smartcards realisiert werden. Die Freigabe kann dabei sowohl lokal an dem Ort, an dem die Datenerfassung und/oder die Behandlung stattfindet als auch an einem anderen Ort über eine digitale Kommunikationseinrichtung stattfinden, so dass im letzteren Fall keine Person außer dem Patienten, beispielsweise kein Arzt und/oder nur eine Assistenzperson bei der Behandlung vor Ort präsent sein muss. Nach der Identifizierung kann eine Person oder ein weiteres System zum Zweck der Freigabe Zugang zu den von der Datenerfassungseinrichtung erfassten Daten und/oder zu von der Prozesssteuereinrichtung erzeugten Steuerungsdaten für die Behandlungseinrichtung erhalten. Es kann dabei auch vorgesehen sein, dass das weitere System oder die identifizierte Person die Möglichkeit zur Änderung von Steuerungsdaten zur Steuerung der Behandlung erhält.

Die Prozesssteuerungseinrichtung kann, wie oben bereits erwähnt, ganz oder teilweise sowohl am Ort des Datenerfassungs- und Behandlungssystems als auch separat und in der Entfernung von diesem angeordnet oder im Falle einer Realisierung in einer Cloud überhaupt nicht lokalisiert sein.

Beispielsweise kann ein Teil der Prozesssteuerungseinrichtung zur Erzeugung von Steuerungsdaten in Form von Behandlungsplänen dienen, während ein anderer Teil der Prozesssteuerungseinrichtung diese Daten empfängt und für die konkrete Durchführung der Ansteuerung der Behandlungseinrichtung verwendet. Die Ansteuerung kann auch eine Regelung, beispielsweise eine Regelung der Gewebetemperatur der Netzhaut oder im Bereich der Netzhaut, umfassen. Eine derartige Regelung kann vorteilhaft durch einen Teil der Prozesssteuerungseinrichtung erfolgen, der direkt in der Nähe der Datenerfassungseirichtung und/oder der Behandlungseinrichtung angeordnet oder in diese integriert ist. Auch eine solche Regelung kann aus er Entfernung und mittels einer Kommunikationsverbindung erfolgen, beispielsweise, wenn diese die Kommunikation in Echtzeit garantiert, wie beispielsweise eine sogenannte "5G"-Verbindung.

Die durch die Prozesssteuerungseinrichtung steuerbare Behandlungseinrichtung kann in einer möglichen Implementierung eine Vorrichtung zur Gewebebehandlung der Netzhaut eines Auges eines Patienten mittels einer steuerbaren Lichtquelle, beispielsweise eines steuerbaren Lasers, umfassen, wobei der Licht- oder Laserstrahl auf verschiedene Behandlungsflächenbereiche der Netzhaut richtbar ist und wobei die Behandlungseinrichtung weiter dazu eingerichtet ist, jeweils innerhalb von einem oder mehreren Behandlungsflächenbereichen die Netzhaut Spot für Spot nacheinander jeweils durch Bestrahlung mit der Lichtquelle für einen kontrollierbaren Zeitraum auf eine Temperatur zwischen 50 und 55 Grad Celsius zu erwärmen.

Gemäß den medizinischen Behandlungsrichtlinien sollen bisher Netzhauterkrankungen im frühen Stadium nicht durch eine Laserbehandlung therapiert werden, da diese in den bekannten Therapieformen Gewebe zerstört oder zumindest schädigt.

Die Behandlungseinrichtung kann beispielsweise ausschließlich oder zusätzlich zu gewebeverändernden weiteren Behandlungsoptionen eine Behandlungsoption ermöglichen, bei der die Temperaturen, die mit ihr erreicht werden, oberhalb der für den Laserbetrieb zulässigen Augensicherheitsschwelle des Laserbetriebs gewählt werden können, jedoch unterhalb von Temperaturen, bei denen unmittelbar oder verzögert eine Gewebeveränderung durch den Laser hervorgerufen wird. Es hat sich herausgestellt, dass durch die Erwärmung in dem oben genannten Temperaturfenster bei einer derart gestalteten Behandlung physiologische Prozesse im Gewebe in Gang gesetzt oder beschleunigt werden, die einen positiven Effekt auf die Gesundheit und Funktionsweise des Zielgewebes haben. Beispielsweise werden Enzyme, Hormone und Botenstoffe ausgeschüttet oder vermehrt ausgeschüttet, die die Gewebefunktionalität verbessern und damit den Fortschritt von Netzhauterkrankungen verhindern oder verlangsamen. Damit kann eine intravitale Injektion von Medikamenten oder eine andere aufwändige Intervention vermieden werden. Eine derartige Behandlung in dem genannten Temperaturfenster kann in vielen Fällen für die Netzhaut oder Teile der Netzhaut automatisiert durchgeführt werden, auch ohne dass eine Unterbrechung des Prozesses zum Zeck einer Freigabe zur Fortsetzung vorgesehen wird. Im Gegensatz dazu kann, wenn zumindest an einer oder mehreren Stellen der Netzhaut, die genannten Temperaturen überschritten oder um einen bestimmten Betrag überschritten werden, von dem Prozesssystem eine Unterbrechung des Prozesses bis zu einer Freigabe vorgesehen werden.

Die Erreichung der gewünschten Temperaturen der Netzhaut kann durch Erfahrungswerte der Bestrahlungsparameter eingestellt werden, die beispielsweise auch durch ein selbstlernendes System in der Prozesssteuerungseinrichtung ermittelt werden können. Es kann jedoch auch eine Regelung der Behandlungsintensität mittels einer nichtinvasiven Echtzeitmessung und Überwachung der Temperatur am jeweiligen Bestrahlungsort durch ein spektroskopisches Verfahren vorgesehen sein, wie es beispielsweise in der Offenlegungsschrift EP1279385A1 beschrieben ist. Dabei wird eine Reaktion des Gewebes auf einen kurzen Strahlungsimpuls in Form von transienten Drucksignalen einer Druckwelle beobachtet, wobei die Signale über ihre Abhängigkeit von dem sogenannten Grüneisenkoeffizienten eine Temperaturbestimmung des Gewebes erlauben. Die Zielgröße ist dabei eine zu erreichende Zieltemperatur des Gewebes und die Stellgröße ist die steuerbare Strahlungsintensität der Lichtquelle oder die Bestrahlungsdauer oder ein Bestrahlungsmodus, der eine Modulation der Lichtquelle oder weitere Größen umfassen kann.

In einer Ausführungsform kann vorgesehen sein, dass die Behandlungseinrichtung dazu eingerichtet ist, die Netzhaut Spot für Spot nacheinander jeweils durch Bestrahlung mit dem Laser für einen Zeitraum von weniger als 500 msec (millisekunden), insbesondere weniger als 200 msec auf eine Temperatur zwischen 50 und 55 Grad Celsius zu erwärmen.

Dabei kann weiter vorgesehen sein, dass der Zeitraum der Bestrahlung mit der genannten Leistung wenigstens 10 msec, weiter insbesondere wenigstens 50 msec, weiter insbesondere wenigstens 100 oder 150 msec beträgt.

Beispielsweise können sich somit Bestrahlungszeiten zwischen 50 und 200 msec oder zwischen 50 und 500 msec oder zwischen 100 und 200 msec oder zwischen 100 und 500 msec oder zwischen 150 und 500 msec ergeben.

Durch Einhaltung eines optimalen Zeitfensters kann einerseits sichergestellt werden, dass eine Zieltemperatur des Gewebes der Netzhaut oder des Gewebes in ihrer unmittelbaren Umgebung in dem Temperaturfenster erreicht wird und dass andererseits kritische Grenztemperaturen nicht überschritten werden, so dass Gewebeschädigungen sicher vermieden werden können.

Die Behandlungseinrichtung und/oder die Prozesssteuerungseinrichtung im Zusammenwirken mit der Behandlungseinrichtung kann auch dazu eingerichtet sein, jeweils innerhalb von einem oder mehreren Behandlungsflächenbereichen die Netzhaut Spot für Spot nacheinander jeweils durch Bestrahlung mit dem Laser für einen Zeitraum von weniger als 500 msec (Millisekunden), insbesondere weniger als 200 msec mit einer Leistungsdichte zwischen 150 W/cm$^2$ und 350 W/cm$^2$, insbesondere zwischen 180 W/cm$^2$ und 300 W/cm$^2$ zu bestrahlen.

Auch in diesem Fall können vorteilhaft die weiter oben genannten Grenzen für die Bestrahlungszeit mit den genannten Leistungen gelten sowie die oben angegebenen Zeitfenster.

Die angegebenen Leistungsdichten des Lasers oder allgemein der Lichtquelle führen dazu, dass das Gewebe für eine geeignete Zeitspanne eine Temperatur erreicht, bei der durch die Erwärmung physiologische Prozesse im Gewebe in Gang gesetzt oder beschleunigt werden, die einen positiven Effekt auf die Gesundheit und Funktionsweise des Zielgewebes haben. Dabei werden Enzyme, Hormone und Botenstoffe ausgeschüttet oder vermehrt ausgeschüttet, die die Gewebefunktionalität verbessern und damit den Fortschritt von Netzhauterkrankungen verhindern oder verlangsamen.

In einer weiteren Ausführungsform kann vorgesehen sein, dass die Behandlungseinrichtung und/oder die Prozesssteuerungseinrichtung im Zusammenwirken mit der Behandlungseinrichtung dazu eingerichtet ist, mit dem Laser zu behandelnde Behandlungsflächenbereiche vordefinierten Flächenabschnitten zuzuordnen und gemäß einer Reihenfolge zu bestrahlen, die von der Zuordnung der Behandlungsflächenbereiche zu den Flächenabschnitten bestimmt ist. Behandlungsflächenbereiche können dabei solche Flächenbereiche der Netzhaut sein, die für eine Behandlung ausgewählt worden sind, beispielsweise, weil dort bestimmte nicht optimale Gewebezustände ermittelt worden sind.

Um für eine Netzhautbehandlung eine optimierte Planung und Ansteuerung des Behandlungslasers im Sinne einer optimierten Logistik zu ermöglichen, sind Flächenabschnitte unabhängig von dem jeweiligen Patienten vordefiniert, denen die Behandlungsflächenbereiche zugeordnet werden können. Damit lassen sich die Behandlungsflächenbereiche, in denen die Netzhaut behandelt werden soll, auf der Netzhaut einfacher, zuverlässiger und besser reproduzierbar bezeichnen und auffinden. Auch der Behandlungslaser lässt sich mit Bezug auf die vordefinierten Flächenabschnitte leichter ansteuern. Die Behandlungsflächenbereiche können frei auf der Netzhaut positioniert werden, jedoch lassen sie sich durch die Zuordnung zu den vordefinierten Flächenabschnitten leicht und schnell auffinden und mittels des Behandlungslasers ansteuern. Ein weiterer Vorteil der Nutzung von vordefinierten Flächenabschnitten liegt darin, dass sie eine Standardisierung für Ortsangaben auf der Netzhaut zulassen, so dass beispielsweise mittels eines ersten Systems Behandlungsflächenbereiche festgelegt und mittels eines anderen Systems und/oder zeitlich versetzt die Positionen der Behandlungsflächenbereiche wieder aufgefunden und angesteuert werden können. Eine bereits bekannte Einteilung solcher vordefinierten Flächenabschnitte ist durch das sogenannte ETDRS- Grid gegeben, das bereits im Bereich der Macula verwendet wird, um diagnostische Informationen, die Ergebnisse von Funktionsprüfungen und beispielsweise auch die Ergebnisse von Schichtdickenmessungen durch optische Kohärenztomografie zu kategorisieren. Es sind jedoch auch andere Einteilungen von Flächenabschnitten denkbar.

Das Datenerfassungssystem und/oder das Prozesssteuerungssystem und/oder das Behandlungssystem können dazu eingerichtet sein, die durch ein solches ETDRS- Grid vordefinierten Flächenabschnitte oder diejenigen Flächenabschnitte, in denen Behandlungsflächenbereiche liegen, jeweils einheitlich zu behandeln. Das kann beispielsweise bedeuten, dass das Datenerfassungssystem nur jedem der vordefinierten Flächenabschnitte einen Mess- Datensatz zuordnet. Eine weitere Möglichkeit kann vorsehen, dass die Prozesssteuerungsvorrichtung jedem der vordefinierten Flächenabschnitte einen einzigen Satz von Steuerungsdaten zuordnet oder dass die Behandlungseinrichtung dazu eingerichtet ist, jeden der vordefinierten Flächenabschnitte einheitlich und mit gleichbleibenden Bestrahlungsparametern zu bestrahlen.

Die Behandlungseinrichtung und/oder die Prozesssteuerungseinrichtung im Zusammenwirken mit der Behandlungseinrichtung kann auch dazu eingerichtet sein, dass der Laser die vordefinierten Flächenabschnitte oder die Behandlungsflächenbereiche in diesen Flächenabschnitten in einer vorgegebenen Reihenfolge oder nach einer vorgegebenen Regel nacheinander bestrahlt.

Durch die Zuordnung der Behandlungsflächenbereiche zu den vordefinierten Flächenabschnitten können die Behandlungsflächenbereiche leicht mit dem Behandlungslaser angesteuert werden. Es lässt sich auch im Vorfeld der Behandlung oder bereits bei der Programmierung der Prozesssteuerungseinrichtung eine Reihenfolge der Bestrahlungen gemäß der Zugehörigkeit zu den Flächenabschnitten festlegen, so dass die Einstellungsänderungen, zum Beispiel Strahlwinkeländerungen, des Lasers von Flächenabschnitt zu Flächenabschnitt und damit auch von einem Behandlungsflächenbereich zum nächsten durch eine Vorausplanung minimiert werden können. Damit lassen sich sowohl Bestrahlungsungenauigkeiten minimieren, als auch die Zeit, die für die Gesamtbehandlung notwendig ist.

Es kann zudem vorgesehen sein, dass die vordefinierten Flächenabschnitte gemeinsam eine die Fovea umgebende, die Fovea ausnehmende Fläche abdecken, deren Ausdehnung derart bemessen ist, dass sie den Austrittsort des Sehnervs und insbesondere die Arcaden nicht abdeckt. Die Flächenabschnitte können dabei die Fovea konzentrisch umgeben, so dass die Fovea das Zentrum der gesamten, durch die Flächenabschnitte abgedeckten Fläche bildet.

Unter den Arcaden soll in diesem Zusammenhang die großen Blutgefäße verstanden werden, die das Sehfeld auf der Netzhaut bogenförmig umgeben und vom Austrittsort des Sehnervs in die Netzhaut ausgehen.

Die Behandlungseinrichtung und/oder die Prozesssteuerungseinrichtung im Zusammenwirken mit der Behandlungseinrichtung kann auch dazu eingerichtet sein, eine Gruppe von Flächenabschnitten, insbesondere alle Flächenabschnitte einzeln nacheinander jeweils entweder nicht oder zu 100% flächendeckend zu bestrahlen.

Da die Laserbehandlung gemäß den oben beschriebenen Regeln im Gewebe der Netzhaut keinen Schaden anrichten kann, ist auch die Bestrahlung von zusätzlichem Gewebe unschädlich, auch wenn sie die Behandlungszeit verlängert. Solange in einem Behandlungsflächenbereich innerhalb eines Flächenabschnitts eine Laserbehandlung nützlich ist, kann daher auch der gesamte Flächenabschnitt bestrahlt, das heißt durch zu bestrahlende Laserspots abgedeckt werden. Dies macht die Behandlungsplanung besonders einfach, da die Grenzen der Flächenabschnitte festliegen und auch Muster zur Abdeckung ganzer Flächenabschnitte mit Laserspots/Bestrahlungsspots vorgefertigt abgespeichert werden können. Flächenabschnitte, in denen keine Behandlungsflächenbereiche liegen, können bei der Bestrahlung übersprungen werden.

Es kann in einer weiteren Ausgestaltung auch vorgesehen sein, dass wenigstens eine Gruppe von Flächenabschnitten in Umfangsrichtung um die Fovea herum nebeneinander angeordnet sind.

Bei einer solchen Anordnung können im Rahmen der Laserbehandlung die einzelnen Flächenabschnitte vollständig oder die auf ihnen angeordneten Behandlungsflächenbereiche nacheinander mit dem Behandlungslaser angefahren und abgearbeitet werden. Dabei können beispielsweise jeweils unmittelbar aneinander angrenzende Flächenabschnitte oder die auf aneinander angrenzenden Flächenabschnitten angeordneten Behandlungsflächenbereiche unmittelbar nacheinander behandelt werden. Die Fovea selbst wird dabei ausgenommen, da sie in den Flächenabschnitten nicht enthalten und durch diese nicht abgedeckt ist.

Eine weitere sinnvolle Ausgestaltung kann vorsehen, dass die Flächenabschnitte um die Fovea herum verteilt sind, insbesondere in der Form von Segmenten wenigstens eines die Fovea konzentrisch umgebenden Ringes oder Kreisrings oder eines die Fovea umgebenden elliptischen Ringes oder dass die Flächenabschnitte auf einem oder mehreren, jeweils die Fovea konzentrisch umgebenden Ringen, insbesondere Kreisringen liegen, wobei die Flächenabschnitte rotationssymmetrisch mit einer n-zähligen Rotationssymmetrie angeordnet sind, wobei n insbesondere zwischen 1 und 9 liegt und weiter insbesondere 2, 4 oder 6 beträgt.

Durch eine rotationssymmetrische Anordnung der Flächenabschnitte mit n-zähliger Symmetrie ergibt sich eine übersichtliche Anordnung und Adressierbarkeit der Flächenabschnitte. Beispielsweise können diese in einem regelmäßigen Vieleck, insbesondere Viereck, Fünfeck oder Sechseck um die Fovea herum verteilt sein. Die Flächenabschnitte können jeweils gleich groß oder auch unterschiedlich groß sein.

Bei dem Prozesssystem, insbesondere in der Behandlungseinrichtung und/oder in der Prozesssteuerungseinrichtung im Zusammenwirken mit der Behandlungseinrichtung kann weiter auch eine eye-tracking-Einrichtung vorgesehen sein, die während der Bestrahlung der Behandlungsbereiche Bewegungen des Auges kompensiert.

Sobald die zu behandelnden Bereiche der Netzhaut festgelegt sind und die Lichtquelle/der Behandlungslaser programmiert ist, kann jede Augenbewegung dazu führen, dass die Netzhaut sich unter dem Behandlungsstrahl lateral bewegt und damit nicht behandlungswürdige Bereiche der Netzhaut oder solche Bereiche, die nicht bestrahlt werden dürfen, dem Licht/Laserstrahl ausgesetzt werden.

Aus diesem Grund wird ein sogenanntes Eye-Tracking durchgeführt, das heißt, die Bewegungen des behandelten Auges werden erfasst und die Lenkung des Strahls der Behandlungslichtquelle/des Behandlungslasers wird derart angepasst, dass die Zielbereiche der Netzhaut trotz der Augenbewegung zuverlässig getroffen werden. Die Augenbewegung kann dabei beispielsweise entweder mittels einer Kamera oder durch die Erfassung von reflektierten Abtaststrahlen erfasst werden.

Das Prozesssystem kann, wie bereits oben angedeutet, derart gestaltet sein, dass die Prozesssteuerungseinrichtung wenigstens zum Teil durch eine von dem Datenerfassungs-und Behandlungssystem gesondert vorgesehene und mit diesem über Kommunikationsverbindungen verbundene Datenverarbeitungseinrichtung gebildet ist, wobei diese Datenverarbeitungseinrichtung insbesondere wenigstens teilweise in Form eines Netzwerks mit mehreren Recheneinrichtungen realisiert ist. Die Prozesssteuerungseinrichtung kann somit wenigstens teilweise als Teil einer cloud realisiert sein.

Dies kann den Vorteil haben, dass eine Prozesssteuerungseinrichtung zentral verwaltet und mit mehreren Datenerfassungs- und Behandlungssystemen zur Prozesssteuerung verbunden sein kann. Hierdurch sind Softwarewartung und die Pflege von Datenbanken, auf die die Prozesssteuerungseinrichtung zugreift, vereinfacht. Zudem können lernende Systeme, die einen Teil der Prozesssteuerungseinrichtung bilden können, zentral weiterentwickelt und trainiert werden. Eine solche zentralisierte Prozesssteuerungseinrichtung kann es auch einer einzelnen Person ermöglichen, die Prüfung und Freigabe verschiedener Behandlungsprozesse an verschiedenen Orten zentral vorzunehmen.

Es ist jedoch auch im Gegensatz dazu möglich, eine Prozesssteuerungseinrichtung vor Ort in der unmittelbaren Nähe mit einer Datenerfassungseinrichtung und einer Behandlungseinrichtung vorzusehen, um eine autonome Einheit zu bilden, die beispielsweise auch in einem einzigen Gerät zusammengefasst sein kann. Zumindest kann vorgesehen sein, dass die Datenerfassungseinrichtung einerseits und die Behandlungseinrichtung andererseits als Teile eines Datenerfassungs- und Behandlungssystems baulich in einem einzigen Gerät integriert sind.

Diese bauliche Vereinigung hat unter anderem den Vorteil, dass der Patient in demselben Gerät, wenigstens teilweise sogar unter Verwendung derselben optischen Einrichtungen, vermessen und behandelt werden kann. Die Justierung der Behandlungslichtquelle, insbesondere die Justierung eines Laserstrahls kann mit der Referenz zur Datenerfassung mittels eines bildgebenden Systems vorgenommen werden, da die Steuerung eines Lichtstrahls mittels der Referenz auf das oder die bildgebenden Systeme möglich ist, deren Position und Orientierung relativ zur Behandlungslichtquelle bekannt ist. Die Behandlung durch die Behandlungslichtquelle kann ebenso in vielen Fällen unter unmittelbarer Kontrolle durch dasselbe bildgebende System erfolgen, mit dem eine Datenerfassung vorgenommen wurde. Durch diese Gestaltung des Prozesssystems und des Verfahrens kann einerseits Zeit gespart werden, andererseits können Fehler durch Verwechslungen von Patienten oder Datensätzen oder Fehljustierungen vermieden werden.

Weiter kann in einer Implementierung des Prozesssystems vorgesehen werden, dass dieses ein Bezahlsystem oder wenigstens Elemente eines Bezahlsystems enthält.

Durch die Integration eines Bezahlsystems oder von Teilen davon in das Prozesssystem kann sichergestellt werden, dass eine Bezahlung einer Behandlung mit minimalem Aufwand möglich wird.

Es kann durch das Prozesssteuerungssystem beispielsweise ein Preis festgesetzt werden und/oder es können Modalitäten für eine Bezahlung festgelegt und in einem Kommunikationsschritt mit einem Patienten oder einer Krankenkasse bestätigt werden. Dadurch kann die konkrete Bezahlung auch nach der Behandlung erfolgen. Die Festsetzung des Preises kann dabei unter Berücksichtigung der von der Datenerfassungseinrichtung erfassten Daten, also einem Ausgangszustand, erfolgen, und/oder unter Berücksichtigung einer notwendigen, gebotenen oder empfohlenen Behandlung.

In einer Ausführungsform kann die Integration von Teilen eines Bezahlsystems durch ein in das Prozesssystem integriertes Preisberechnungsmodul realisiert sein, das dazu eingerichtet ist, unter Berücksichtigung der durch die Datenerfassungseinrichtung erfassten Daten und/oder unter Berücksichtigung der durch die Prozesssteuerungseinrichtung ermittelten Steuerungsdaten einen Preis für eine Behandlung zu ermitteln und eine Zahlungsaufforderung auszugeben, wobei das Prozesssystem insbesondere weiter dazu eingerichtet ist, die Erzeugung oder Übermittlung von Steuerungsdaten und/oder die Behandlung zu verzögern, bis ein Bezahlvorgang validiert worden ist.

Zunächst kann von dem Prozesssystem ein zu zahlender Preis an dem Datenerfassungs- und Behandlungssystem, das heißt, dort, wo sich der Patient befindet ausgegeben werden, so dass der Patient einen Preis wahrnehmen und bezahlen kann. Die Bezahlung kann auf elektronischem Weg, beispielsweise durch Ermächtigung eines SEPA- Systems oder durch Verwendung einer Kreditkarte, beispielsweise mittels einer Ermächtigung durch ein Near-field-Kommunikationssystem erfolgen.

Der ermittelte Preis kann jedoch auch von dem Prozesssystem auf elektronischem Weg an eine Krankenkasse zur Zahlung übermittelt werden. Die Krankenkasse oder eine Bezahlstelle hat dann die Möglichkeit, ebenso wie der Patient vor Ort, die Bezahlung unmittelbar vorzunehmen oder zumindest anzuweisen oder zu bestätigen.

Das Prozesssystem kann beispielsweise ein Abrechnungsmodul aufweisen, das dazu eingerichtet ist, Bezahldaten von dem Benutzer und/oder von einem Bezahlinstitut zu empfangen, die Bezahldaten zu validieren und nach erfolgreicher Validierung die Behandlung freizugeben oder zu starten.

Das Prozesssystem kann beispielsweise auch dazu eingerichtet sein, mit einem mobilen Endgerät zusammenzuwirken und von diesem wenigstens Daten einer der folgenden Kategorien zu empfangen und zu verarbeiten:

Patientenidentifikationsdaten

Messdaten von Messungen, die mittels des Endgerätes durchgeführt werden,

Bezahldaten.

Das Austauschen von Bezahldaten ist weiter oben bereits angesprochen worden. Das mobile Endgerät, beispielsweise ein Smartphone, kann in zertifizierter und abgesicherter Weise eine Zahlungsanweisung bestätigen, so dass die Prozesssteuerungseinrichtung von einem Bezahlsystem, mit dem es kommuniziert, die Bezahlung verlangen und erhalten oder bestätigt bekommen kann.

Mittels des Endgeräts können auch Patientenidentifikationsdaten an das Prozesssystem übermittelt werden. Diese können persönliche Daten wie beispielsweise Namen, Adresse, Alter, Größe, Haarfarbe, Versicherungsnummern, Name der Krankenversicherung, Telefonnummer sowie Gesundheitsdaten wie bereits vorgenommene Behandlungen oder die Referenz zu in Datenbanken gespeicherten Gesundheitsdaten und/oder die Referenz zu bereits früher mit dem Prozesssystem erfasste Daten, Netzhautaufnahmen oder gespeicherte Netzhautbehandlungsparameter sein.

Die Messdaten von Messungen, die mittels des Endgerätes durchgeführt werden, können Testergebnisse von Sehtests sein, die mit dem Endgerät vorgenommen worden sind und durch die beispielsweise die Sehschärfe oder die Fähigkeit zur Wahrnehmung von Kontrasten gemessen werden können. Mittels des Endgerätes können jedoch auch unmittelbar mit einer Kamera Bildaufnahmen von Haut, Haaren und der Iris des Patienten gemacht werden oder auch Bilder von der Netzhaut des Patienten. Diese Netzhautbilder können teilweise auch bestimmte technische Ergänzungselemente wie Zusatzlinsen oder Adapterstücke erfordern. Auch diese Daten können zur Vorinformation des Prozesssystems dienen oder für eine erste Prüfung verwendet werden, ob eine Behandlung möglich, notwendig oder empfehlenswert ist. Zum Zweck einer solchen Prüfung kann ein Analyseprogramm in dem mobilen Endgerät installiert sein oder das Endgerät kann derartige Daten über eine Kommunikationsschnittstelle an das Prozesssteuerungssystem zur Prüfung übermitteln und erste Auswertungen erhalten.

Das Prozesssystem kann dann beispielsweise dazu eingerichtet sein, die Datenerfassungseinrichtung zur Erfassung wenigstens einer Aufnahme der Netzhaut in Abhängigkeit von durch das mobile Endgerät an das Prozesssystem übermittelten Patientenidentifikationsdaten und/oder von Messdaten von Messungen zu steuern, die mittels des Endgerätes durchgeführt wurden.

Durch eine Vorprüfung des Patienten kann einerseits vermieden werden, dass Patienten zur Behandlung erscheinen, für die zumindest eine automatisierte Behandlung nicht hilfreich oder möglich ist. Andererseits kann für solche Patienten, für die eine automatisierte Behandlung möglich ist, der für die Datenerfassung benötigte Zeitaufwand verringert werden.

Es kann weiter vorgesehen sein, dass die Prozesssteuerungseinrichtung die Steuerungsdaten zur Steuerung der Behandlungseinrichtung in Abhängigkeit von durch das mobile Endgerät an das Prozesssystem übermittelten Patientenidentifikationsdaten und/oder von Messdaten von Messungen erzeugt, die mittels des Endgerätes durchgeführt wurden.

In manchen Fällen kann demnach bereits die Datenerfassung durch das mobile Endgerät für die Festlegung von Behandlungsoptionen oder- Parametern ausreichen. In anderen Fällen kann jedoch auch der Umstand genutzt werden, dass die Daten, die mittels des mobilen Endgerätes erfasst wurden, eine Ergänzung zu den Daten darstellen, die mittels der Datenerfassungseinrichtung des Prozesssystems erfasst werden. Einerseits können durch die beiden Varianten der Datenerfassung unterschiedliche Messgrößen erfasst werden, andererseits können auch gleiche oder ähnliche Messgrößen zu unterschiedlichen Zeiten erfasst und registriert werden, so dass entweder eine Bestätigung bestimmter Messergebnisse ermöglicht wird oder eine Einschätzung von Zustandsänderungen beispielsweise der Netzhaut, da diese zu unterschiedlichen Zeitpunkten mittels des mobilen Endgerätes und mittels der Datenerfassungseinrichtung des Prozesssystems erfasst worden ist.

Es kann weiter in einer möglichen Implementierung vorgesehen sein, dass ein mögliches und durch die Prozesssteuerungseinrichtung auswählbares Behandlungsverfahren eine Behandlung der Netzhaut mit einer Bestrahlungsintensität vorsieht, die im Gewebe der Netzhaut Temperaturen von weniger als 58 Grad Celsius, insbesondere mit einer Höchsttemperatur zwischen 48Grad und 58 Grad, weiter insbesondere mit einer Höchsttemperatur zwischen 50 Grad und 55 Grad erzeugt.

Eine derartige Behandlung ist bei einer Mehrzahl der Patienten gefahrlos möglich, auch wenn sie im Einzelfall nicht notwendig sein sollte.

Andere Optionen des Prozesssystems können Bestrahlungsmodalitäten mit einer zumindest abschnittsweise intensiveren Bestrahlung sein, die auch teilweise gewebeverändernd sein kann. Eine weitere Option für das Prozesssteuerungssystem kann die Ablehnung einer Behandlung sein, weil diese nicht notwendig oder nicht hilfreich ist oder die Empfehlung für den Patienten, einen Experten zur näheren Untersuchung und/oder zur nicht automatisierten Behandlung aufzusuchen.

Die Erfindung bezieht sich außer auf ein Prozesssystem der oben beschriebenen Art auch auf ein Datenerfassungs-und Behandlungssystem zur Behandlung der Netzhaut eines Auges mittels einer Behandlungslichtquelle, insbesondere eines Behandlungslasers, mit

einer Datenerfassungseinrichtung, die wenigstens eine Aufnahme der Netzhaut erfasst und mit einer Behandlungseinrichtung zur Behandlung der Netzhaut mit der Behandlungslichtquelle in Abhängigkeit von der wenigstens einen Aufnahme der Netzhaut, wobei die Datenerfassungseinrichtung dazu eingerichtet ist, erste Daten mittels einer Kommunikationsverbindung an eine Prozesssteuerungseinrichtung zu senden und wobei die Behandlungseinrichtung dazu eingerichtet ist, zweite Daten mittels einer Kommunikationsverbindung von der Prozesssteuerungseinrichtung zu empfangen, wobei das Datenerfassungs- und Behandlungssystem dazu eingerichtet ist, mit einem mobilen Endgerät, insbesondere einem Mobiltelefon über eine Funkschnittstelle Daten auszutauschen, die eine Reichweite von weniger als 30 m, insbesondere weniger als 15 m hat, insbesondere eine Bluetooth-, WLAN- oder NFC- Schnittstelle und/oder wobei das Datenerfassungs- und Behandlungssystem eine Bezahlschnittstelle aufweist.

Bei einer derartigen Gestaltung kann das Datenerfassungs-und Behandlungssystem einerseits unabhängig von einer Prozesssteuerungseinrichtung zur Vorbereitung einer Behandlung durch eine geeignete Datenerfassung dienen und ebenso zur Durchführung einer Behandlung, die mit Steuerungsdaten gesteuert ist, die entweder durch eine Prozesssteuerungseinrichtung der oben beschriebenen Art erzeugt worden oder durch einen Experten/Arzt festgelegt sind. Weiter kann das Datenerfassungs-und Behandlungssystem davon profitieren, dass ihm Daten von dem mobilen Endgerät übermittelt werden können, die der Ergänzung oder Bestätigung der Datenerfassung durch die Datenerfassungseinrichtung dienen können. Der Austausch von Daten mit dem mobilen Endgerät ist dabei unproblematisch und weitgehend abhörsicher oder kann zumindest mit der gewünschten Datensicherheit ausgestaltet werden, beispielsweise durch eine end-to-end-Verschlüsselung. Mit dem Endgerät können außer Messdaten beispielsweise auch Bezahldaten unmittelbar ausgetauscht und es kann eine Bezahlung vorgenommen werden.

Weiter bezieht sich die Erfindung auf ein Verfahren zur Behandlung der Netzhaut eines Auges mit einem Prozesssystem mit einem Datenerfassungs-und Behandlungssystem, das eine Datenerfassungseinrichtung und eine Behandlungseinrichtung zur Behandlung der Netzhaut mit einer Behandlungslichtquelle in Abhängigkeit von der wenigstens einen Aufnahme der Netzhaut sowie eine Prozesssteuerungseinrichtung umfasst, wobei die Datenerfassungseinrichtung wenigstens eine Aufnahme der Netzhaut erfasst und an die Prozesssteuerungseinrichtung übermittelt und die Prozesssteuerungseinrichtung in Abhängigkeit von den durch die Datenerfassungseinrichtung erfassten Daten selbsttätig Steuerungsdaten zur Steuerung einer Behandlungseinrichtung erzeugt, und wobei das Verfahren vor Beginn der Behandlung durch die Behandlungseinrichtung unterbrochen und nur nach einer Freigabe selbsttätig fortgesetzt wird.

Bekannte Verfahren sind zum Teil teilautomatisiert, decken jedoch in jedem Fall nur Teilabschnitte des Gesamtprozesses ab, da davon ausgegangen wird, dass entweder die Datenerfassung fehlerbehaftet sein kann oder die Behandlung derart kritisch ist, dass ein Experte in Form eines Augenarztes involviert sein muss. Gemäß der vorliegenden Erfindung wird in einem Gesamtprozess sichergestellt, dass ein Experte nur zur Überprüfung und Freigabe von Steuerungsdaten zum Beginn der Behandlung eingeschaltet wird, so dass sich zumindest für einige Patienten eine deutliche Verringerung des Aufwandes einstellt.

Auch wenn für einige Patienten eine Freigabe durch einen Experten abgewartet werden muss, ergeben sich auch für diese Patienten Vorteile durch die umfassende und automatisierte Zusammenarbeit und Datenkommunikation des Datenerfassungs- und Behandlungssystems, der Prozesssteuerungseinrichtung und optional durch den Austausch von Daten mit einem mobilen Endgerät und die Integration zumindest von Teilen eines Bezahlvorganges. Die erfassten und ermittelten Daten werden selbsttätig zwischen den verschiedenen Teilen des Prozesssystems elektronisch übermittelt und jeweils bearbeitet. Auch die Freigabe kann elektronisch erfolgen. Die Freigabe sowie die Identität der freigebenden Person kann auch, ebenso wir die erfassten Aufnahmen der Netzhaut und die erzeugten Steuerungsparameter, zur Dokumentation im Prozesssystem aufgezeichnet werden. Dies ermöglicht und erleichtert dann eine Nachsorge und/oder Nachbehandlung.

Im Folgenden wird die Erfindung anhand von Ausführungsbeispielen in Figuren einer Zeichnung gezeigt und anschließend beschrieben.

Dabei zeigt

Figur 1: eine Vorrichtung zur Gewebebehandlung der Netzhaut mit einem Laser,

Figur 2: eine Behandlungsvorrichtung mit einem Behandlungslaser und mit einer Temperaturkontrolle,

Figur 3: ein Diagramm über besondere Leistungsdichtebereiche für die Netzhautlaserbehandlung,

Figur 4, 5, 6: vordefinierbare Flächenabschnitte der Netzhaut,

Figur 7: Flächenabschnitte mit einem Behandlungsbereich und Laserspots, die sich im Behandlungsbereich befinden,

Figur 8: ein Gerät zur Laserbehandlung der Netzhaut mit einer Datenerfassungseinrichtung in Form einer Bildgebungseinrichtung,

Figur 9: eine schematische Darstellung eines beispielhaften Prozesssystems,

Figur 10: eine schematische Darstellung eines weiteren Prozesssystems mit einer Kopplung an ein mobiles Endgerät,

Figur 11: eine schematische Darstellung eines Prozesssystems, bei dem ein mobiles Endgerät lokal an ein Datenerfassungs- und Behandlungssystem gekoppelt ist, sowie

Figur 12: eine schematische Darstellung von Verfahrensschritten.

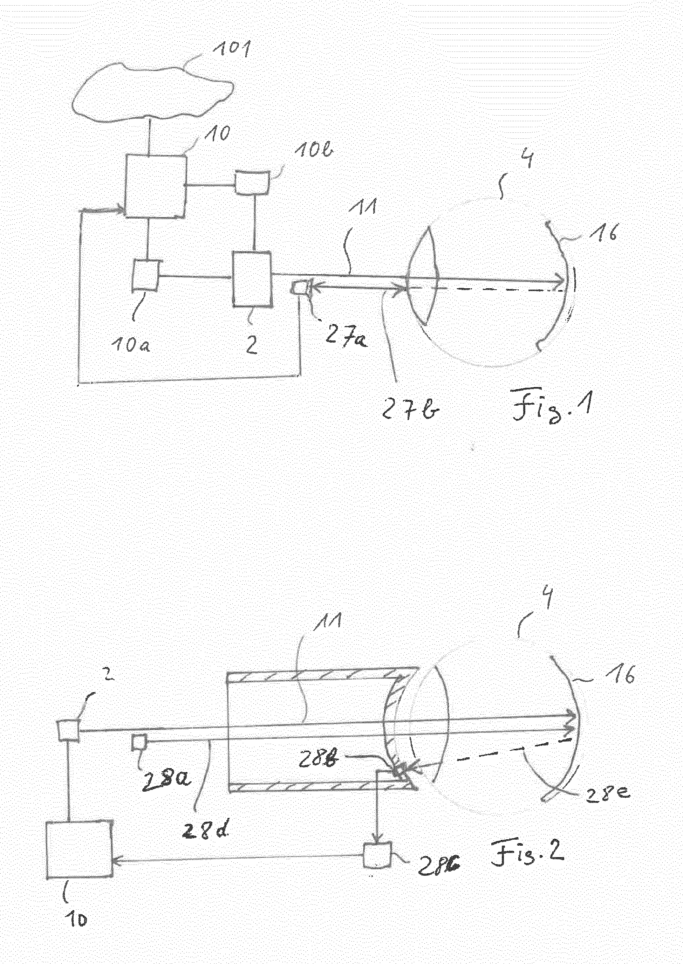

Figur 1 zeigt eine Vorrichtung zur Gewebebehandlung der Netzhaut 16 eines Auges 4 eines Patientenmit einem Laser/Behandlungslaser 2, der einen Laserstrahl 11 durch die Linse des Auges auf die Netzhaut sendet. Anstelle eines Lasers kann in einigen Fällen auch eine andere Lichtquelle verwendet werden, z. B. eine Leuchtdiode. Eine Prozesssteuerungseinrichtung 10 steuert im Allgemeinen die Richtung und Intensität der Laserstrahlung und in einigen Fällen konkret Parameter wie die Laserleistung, die Modulationsfrequenz, das Tastverhältnis des Lasers, die Laserkollimation, die Größe des Laserspots und in einigen Fällen auch die Wellenlänge der Laserstrahlung. Diese Parameter können von der Prozesssteuerungseinrichtung 10 in Abstimmung mit der spezielleren und potenziell direkt mit dem Laser verbundenen Steuereinheit 10a des Lasers gesteuert werden. Ferner kann die Ablenkung des Laserstrahls über eine Ablenkungssteuereinheit 10b gesteuert werden, die in einigen Fällen einen oder mehrere Ablenkungsspiegel, beispielsweise MEMS- Spiegel, oder andere vergleichbare Ablenkungsmittel steuert. Die Ablenkungssteuereinheit 10b ermöglicht eine Abtastung oder das Scannen der Netzhautoberfläche, beispielsweise durch ein Laserspotmuster mit einem Laserspot nach dem anderen. Eine Bestrahlung eines Punktes nach dem anderen kann bedeuten, dass der Lichtstrahl von Punkt zu Punkt springt und für eine bestimmte Zeit auf jedem Punkt verbleibt, sie kann aber auch, in einigen Ausführungsformen der Erfindung, eine kontinuierliche Bewegung von einem Punkt zum nächsten oder eine Bewegung umfassen, bei der sich schnellere und langsamere Bewegungsphasen des Laserspots abwechseln.

Die Prozesssteuerungseinrichtung 10 ist mit einem Computernetzwerk/Cloud 101 und/oder einem entfernten Server verbunden oder in diese integriert, um Daten mit anderen Datenquellen auszutauschen, zum Beispiel Daten des zu behandelnden Patienten mit einer Datenbank. Beispielsweise können Daten über die Netzhaut des Patienten oder andere physiologische Daten oder persönliche Daten im Computernetzwerk gespeichert werden, zusammen mit einer Freigabe für eine allgemeine Behandlung mit oder ohne besondere Bedingungen. Darüber hinaus können Teile der möglicherweise erforderlichen Berechnungen sowie jegliche Art von Software-Updates in der Cloud vorgenommen werden.

Die Vorrichtung oder allgemeiner das Prozesssystem umfasst ferner eine Eye-Tracking-Einrichtung 27a, 27b mit einer auf das Auge gerichteten Kamera 27a, die zur Beobachtung von Bewegungen des Auges eingerichtet ist. Zu diesem Zweck kann in einigen Fällen ein Lichtstrahl 27b verwendet werden, der auf das Auge gerichtet ist und an der Oberfläche des Auges reflektiert wird, wobei die Reflexion von der Kamera 27a und einer Analysevorrichtung beobachtet und analysiert wird. Die Informationen über die Augenbewegung werden dann an die Prozesssteuerungseinrichtung 10 gesendet, die die Ablenkung des Laserstrahls 11 des Behandlungslasers entsprechend anpassen kann, um den Laserstrahl auf einem Zielpunkt zu halten. Die in der Figur 1 gezeigte Vorrichtung kann einen Teil eines erfindungsgemäßen Prozesssystems bilden. Das Prozesssystem umfasst zusätzlich zu den in der Figur 1 gezeigten Elementen in jedem Fall noch eine Datenerfassungseinrichtung.

Figur 2 zeigt eine ähnliche Behandlungseinrichtung wie Figur 1, wobei die Lasersteuerung vereinfacht dargestellt wurde und nur ein Laser 2 gezeigt wird, der von einer Prozesssteuerungseinrichtung 10 gesteuert wird. Ein Laserstrahl ist auf die Netzhaut 16 eines Auges 4 gerichtet, um z. B. eine Makula-Degenerationskrankheit zu behandeln.

Eine Steuerung der Temperatur, die durch die Behandlung auf der Netzhaut erreicht wird, soll sicherstellen, dass die Gewebetemperatur der Netzhaut oder in der unmittelbaren Umgebung der Netzhaut sehr zuverlässig in einem bestimmten begrenzten Temperaturfenster bleibt. Dies wird in vielen Fällen durch die Steuerung der Laserleistung sowie anderer Parameter des Lasers erreicht. Die mögliche Absorption des Laserlichts durch die Netzhaut kann besser beurteilt werden, wenn die Netzhaut zusätzlich vor der Behandlung durch eine Aufnahme mit einer Kamera oder einem Scanner analysiert wird. Diese Methode wird weiter unten ausführlicher beschrieben.

Eine weitere Methode zur Einhaltung der Temperaturgrenzen kann durch die Messung der Temperatur der Netzhaut am oder in unmittelbarer Nähe des aktuellen Laserspots und die Steuerung der Laserleistung oder anderer Laserparameter auf der Grundlage dieser Messung oder Information realisiert werden. Mit Hilfe der Temperaturmessung kann ein geschlossener Regelkreis eingerichtet werden. Dieser Aufbau ist in Figur 2 einschließlich der Prozesssteuerungseinrichtung 10 dargestellt.

Die Vorrichtung umfasst zu diesem Zweck einen Gewebetemperaturregelmechanismus mit einer Gewebetemperaturmessvorrichtung 28a, 28b, 28c, die Temperaturmesswerte des Netzhautgewebes an die Prozesssteuerungseinrichtung 10 sendet. Auf diese Weise kann ein geschlossener Regelkreis aufgebaut werden, der dafür sorgt, dass die Zieltemperaturen erreicht und eingehalten werden können. Das Temperaturmessgerät basiert auf der "Grüneisen"-Formel und misst die Absorption kurzer Strahlungsimpulse, die von einem Anregungslaser 28a auf die Netzhaut gesendet werden, durch Antwortsignale in Form von Druckwellen 28e, die durch den plötzlichen Temperaturanstieg auf der Netzhaut erzeugt werden, der durch die Strahlungsimpulse 28d des Anregungslasers hervorgerufen wird. Die Druckwellen werden von einem Druckwandler 28b aufgefangen. Die von dem Druckwandler 28b erzeugten Signale werden von einer Transformationseinheit 28c in Temperaturwerte umgewandelt.

Bei dem Anregungslaser 28a kann es sich um einen vom Behandlungslaser getrennten Laser handeln, in einigen Ausführungsformen kann aber auch der Behandlungslaser selbst als Anregungslaser für die zur Temperaturmessung ausgesandten Strahlungsimpulse 28d verwendet werden.

Die Temperaturmesseinrichtung kann dazu verwendet werden, einen geschlossenen Regelkreis mit der Prozesssteuerungseinrichtung 10 aufzubauen und die Temperatur an einem aktuellen Laserspot zuverlässig auf die Zieltemperatur zu begrenzen. Dazu kann die Prozesssteuerungseinrichtung 10 anhand der gemessenen Temperatur die Laserleistung, das Tastverhältnis, den Laserspotdurchmesser oder auch die Wellenlänge des Behandlungslasers steuern.

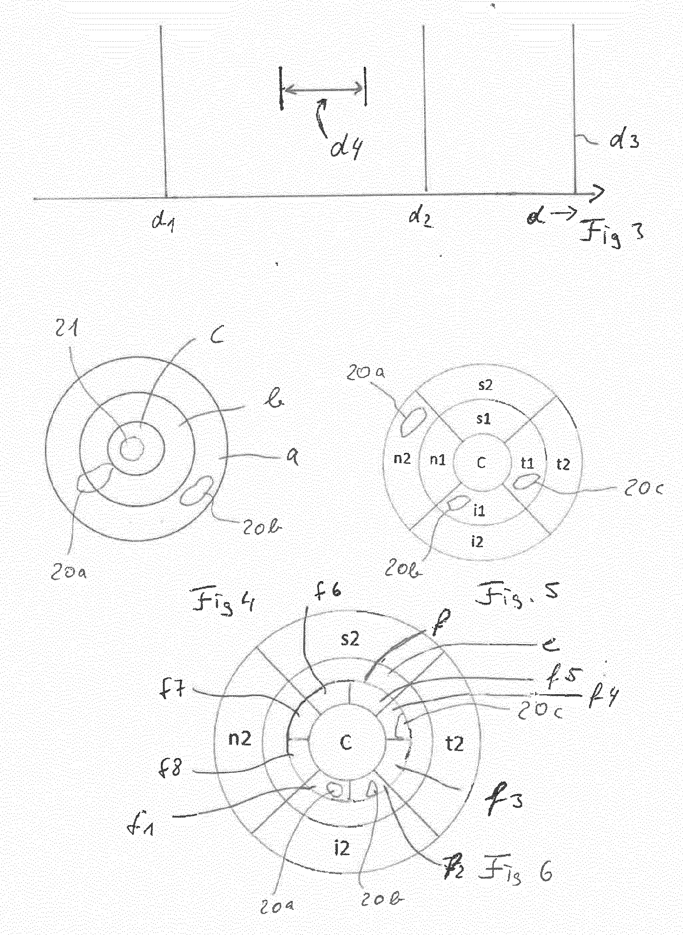

Figur 3 zeigt ein Diagramm mit verschiedenen Bereichen der Laserenergiedichte d oder Strahlungsintensität, die auf der horizontalen Achse aufgetragen ist.

Die erste vertikale Linie beim Wert d1 stellt den offiziellen Grenzwert für die Augensicherheit dar, der in der IEC-Norm IEC 60825-1 mit einem Wert von 31 W/cm2 definiert ist.

Laserleistungsdichten unter diesem Grenzwert sind offiziell ohne jedes Risiko für das Netzhautgewebe anwendbar.

Die zweite vertikale Linie stellt d2 dar, also eine Leistungsdichte von deutlich über 350 W/cm2. Oberhalb dieses Wertes kann die Laserbehandlung leichte Schäden im Netzhautgewebe verursachen, die mit empfindlichen diagnostischen Methoden nachgewiesen werden können, aber in vielen Fällen vom Patienten selbst nicht sofort wahrgenommen werden.

Die dritte vertikale Linie stellt den Wert d3 dar. Laserbehandlungen mit einer Leistungsdichte oberhalb dieses Wertes führen zu einer unmittelbaren Schädigung der Netzhaut, die mit Standardmethoden wahrnehmbar ist.

Der Bereich d4 der Leistungsdichten liegt zwischen d1 und d2. In diesem Bereich wird durch die Laserstrahlung keine Schädigung des Gewebes verursacht, jedoch werden nützliche physiologische Prozesse im Netzhautgewebe ausgelöst, verstärkt, beschleunigt oder zumindest positiv beeinflusst. Dieser Bereich kann bei einer Zieltemperatur des Netzhautgewebes zwischen 48Grad und 58 Grad, insbesondere mit einer Höchsttemperatur zwischen 50 Grad und 55 Grad eingehalten werden.

Die Figuren 4 bis 6 zeigen unterschiedliche Einteilungen von Flächenabschnitten auf der Netzhaut eines Auges. Figur 4 zeigt lediglich zwei konzentrische Kreisringe a und b, die eine zentrale Kreisfläche c umgeben. Die Ringe a und b können Flächenabschnitte der Netzhaut sein, während die Fläche c nicht von den Flächenabschnitten bedeckt ist und nicht bestrahlt werden soll, da sich in dieser zentralen Fläche die Fovea 21 befindet. Es sind zwei Behandlungsflächenbereiche 20a, 20b dargestellt, wobei sich der Behandlungsflächenbereich 20b im Flächenabschnitt a befindet und wobei der Behandlungsflächenbereich 20a sich teilweise auf beide Flächenabschnitte a und b erstreckt.

Figur 5 zeigt das in der Augenheilkunde bekannte Standard-ETDRS-Gitter mit einem Außenkreis, der vier Ringsegmente bildende Flächensegmente i2, n2, s2, t2 aufweist, und mit einem Innenkreis, bei dem die vier Flächensegmente i1, n1, s1, t1 ebenfalls Ringsegmente sind. Figur 5 zeigt ferner drei Behandlungsflächenbereiche 20a, 20b und 20c, wobei sich der Behandlungsflächenbereich 20a im Flächenabschnitt n2, der Behandlungsflächenbereich 20b im Flächenabschnitt i1 und der Behandlungsflächenbereich 20c im Flächenabschnitt t1 befindet.

Figur 6 zeigt eine andere mögliche Zusammensetzung von Flächenabschnitten, wobei ein äußerer kreisförmiger Ring ähnlich der in Figur 5 gezeigten Zusammensetzung in Segmente unterteilt ist, die Flächenabschnitte i2, n2, s2, t2 bilden, während der innere Ring in zwei kleinere konzentrische Ringe e und f unterteilt ist. Der äußere Ring e ist in vier Flächenabschnitte ähnlich den Segmenten der Abschnitte i2, n2, s2, t2 unterteilt, während der innere Ring f in acht Ringsegmente f1, f2, f3, f4 f5, f6, f7, f8 unterteilt ist, die jeweils ein Flächensegment bilden.

Durch diese feinere Unterteilung sind die foveanahen Flächenabschnitte kleiner und somit können in diesem foveanahen Bereich, der sehr empfindlich auf potentielle Schäden reagiert, die zu behandelnden Behandlungsflächenbereiche durch Zuordnung zu Flächenabschnitten genauer bzw. detaillierter lokalisiert werden.

Im Beispiel der Figur 6 sind die Behandlungsflächenbereiche 20a, 20b und 20c den Flächenabschnitten f1, f2 und f4 zugeordnet.

Eine Regel für die Bestrahlung der Behandlungsflächenbereiche kann vorsehen, dass die Behandlungsflächenbereiche beginnend bei f1 entgegen dem Uhrzeigersinn um die Fovea umlaufend behandelt werden. Eine mögliche Regel kann auch besagen, dass alle diejenigen Flächenabschnitte, die Behandlungsflächenbereiche enthalten, jeweils auf ihrer vollen Fläche bestrahlt werden, ebenfalls umlaufend gegen den Uhrzeigersinn oder im Uhrzeigersinn. Eine Regel kann auch besagen, dass für den Fall, dass sich zwischen zwei zu bestrahlenden Flächenabschnitten (im Beispiel von Figur 6 die Flächenabschnitte f2 und f4) nur ein einziger Flächenabschnitt befindet, der keinen Behandlungsflächenbereich aufweist (im Beispiel von Figur 6 der Flächenabschnitt f3), dieser Flächenabschnitt nicht ausgelassen und trotzdem bestrahlt wird. Befinden sich jedoch zwischen zwei Flächenabschnitten mit Behandlungsflächenbereichen mindestens zwei Flächenabschnitte, die keine Behandlungsflächenbereiche tragen, also nicht zur Bestrahlung vorgesehen sind, werden diese Gebietsabschnitte übersprungen.

Durch diese Regel wird ein zügiges Vorgehen erreicht und nur in Einzelfällen werden zusätzliche Flächenabschnitte bestrahlt, die keine Behandlungsflächenbereiche enthalten.

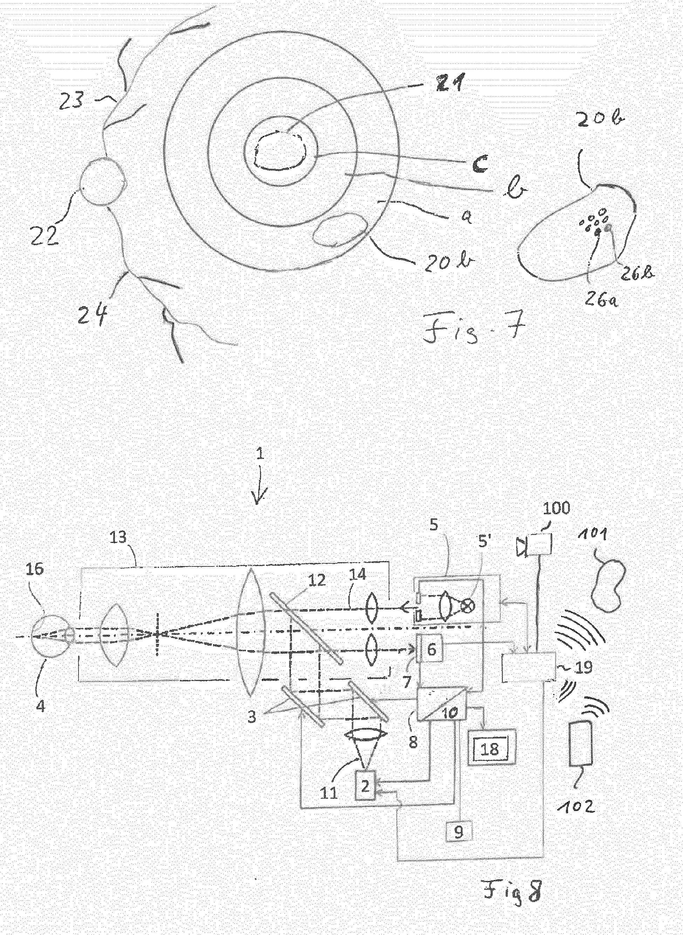

Figur 7 zeigt einen allgemeinen Aufbau von Flächenabschnitten, die einen Teil der Netzhaut abdecken.

Auf der linken Seite der Figur 7 ist der Kopf des optischen Nervs 22 an der Stelle dargestellt, an der er in die Netzhaut eintritt. An dieser Stelle beginnen auch die großen/primären Arterien, die Arkaden 23, 24, die die Netzhaut mit Blut versorgen. Der Bereich der Netzhaut, der in Figur 7 von den Flächenabschnitten a, b abgedeckt wird, wird durch die Fovea 21, die sich in der zentralen Kreisfläche c befindet, und durch die Arkaden 23, 24 und den Kopf des Sehnervs 22 begrenzt und definiert, die sich außerhalb des äußeren Rings a und damit außerhalb der definierten Flächenabschnitte befinden.

Auf der rechten Seite von Figur 7 ist beispielhaft dargestellt, wie ein Behandlungsflächenbereich 20b durch mehrere Laserpunkte 26a, 26b abgedeckt wird, die ein regelmäßiges Muster bilden. Die Muster können im Voraus festgelegt werden, z. B. für jeden der Flächenabschnitte, da ihre Form bekannt und unabhängig von der individuellen Retina ist.

Figur 8 zeigt eine Vorrichtung in Form eines Ophthalmoskops oder eine Vorrichtung, die Funktionen eines Ophthalmoskops mit Funktionen der Laserbehandlung der Netzhaut 16 eines menschlichen Auges 4 kombiniert. Diese Vorrichtung kann somit ein Datenerfassungs- und Behandlungssystem ganz oder teilweise umfassen.

Die Vorrichtung weist neben der Behandlungseinrichtung der Vorrichtung mit dem Laser 2 und den steuerbaren Umlenkspiegeln 3, die für eine allgemeine und ggf. standardisierte Behandlung notwendig sind, eine Datenerfassungseinrichtung auf. Diese weist eine Beleuchtungseinrichtung 5 mit einer Strahlungsquelle 5' auf, die dazu eingerichtet ist, einen Beleuchtungsstrahl 14 auf das Auge 4 und die Netzhaut 16 eines Patienten zu richten. Dadurch kann die Netzhaut 16 für die Aufnahme eines Bildes oder eines Kamerabildes unter standardisierten und reproduzierbaren Bedingungen geeignet beleuchtet werden. Dies ermöglicht in einigen Fällen der Laserbehandlung, nach Überprüfung und Verarbeitung der Netzhautaufnahme durch eine Prozesssteuerungseinrichtung, entweder Standard-Behandlungsparameter für die Behandlung in Form einer Bestrahlung zu verwenden oder die Standard-Behandlungsparameter für einen Patienten entsprechend einer individuellen Messung innerhalb bestimmter Grenzen zu modifizieren.

Die Beleuchtung für die Netzhautaufnahme kann z.B. mit einer Leuchtdiode oder einer Infrarotdiode als Lichtquelle ausgestattet sein, oder mit einer Lichtquelle anderer Art, die ein definiertes Wellenlängenspektrum liefert. Die Lichtquelle kann z. B. auch eine UV-Lichtquelle sein. Als Geräte zur Aufnahme von Bildern der Netzhaut kommen beispielsweise auch Infrarotkameras, Scanner und OCT- Geräte in Frage oder Einrichtungen für eine Autofluoreszenzaufnahme.

Das dargestellte Ophthalmoskop verfügt konkret über eine Kamera 6, in der ein Sensor 7 vorgesehen ist. Der Sensor kann z. B. ein CCD- oder CMOS-Sensor sein. Anstelle der Kamera 6 kann, wie oben anhand konkreter Beispiele aufgezählt, auch jede andere Art von Aufnahmevorrichtung vorgesehen sein, z. B. eine Abtastvorrichtung, die geeignet ist, von der Netzhaut ausgesandte, reflektierte oder gestreute Strahlung zu erfassen.

Ziel des Betriebs der Beleuchtungseinrichtung 5 und der Kamera 6 oder einer gleichwertigen Einrichtung ist es, insbesondere unter definierten Beleuchtungsbedingungen möglichst genaue, ortsaufgelöste Messdaten von der Netzhaut 16 durch Erfassung einer Aufzeichnung der ausgesandten oder rückgestreuten Strahlung zu erhalten und damit die Lage und die Eigenschaften der zu behandelnden Zielbereiche erkennen oder bestimmen zu können.

Die Netzhaut kann auch während der Bestrahlung mit dem Behandlungslaser 2 mittels der Aufnahmeeinrichtung laufend in Echtzeit beobachtet werden, um die Bilder, die in Vorbereitung der Bestrahlung aufgenommen wurden, mit dem Fortgang der Behandlung und der Lenkung des Laserstrahls und der Platzierung der Laserspots abzugleichen. Dies bietet sich besonders dann an, wenn die Bildaufnahmeeinrichtung der Datenerfassungseinrichtung fest und genau definiert relativ zu der Behandlungseinrichtung montiert und orientiert ist.

Der Beleuchtungsstrahl 14 und die reflektierte Strahlung 15 werden durch ein geeignetes optisches System 13 mit Spiegeln und Linsen in bekannter Weise kollimiert und fokussiert. Das optische System 13 verfügt auch über einen Strahlteiler 12, der es ermöglicht, einen Laserstrahl des Behandlungslasers 2 auf die Netzhaut 16 zu richten, wobei die gleiche optische Achse wie für den Beleuchtungsstrahl der Aufnahmeeinrichtung der Datenerfassungseinrichtung verwendet wird. Alternativ kann der Laserstrahl auch ohne Strahlteiler eingekoppelt werden, indem er zum Beispiel leicht seitlich versetzt zum Beleuchtungslicht geführt wird. Zur Steuerung des Lasers 2 kann eine Steuereinheit 8 vorgesehen sein, die Teil einer Prozesssteuerungseinrichtung 8, 19 ist, wobei die Steuereinheit 8 einerseits die Beleuchtungseinrichtung 5 steuert, z.B. auslöst, und andererseits ein Kamerabild von der Kamera 6 erfasst und über eine Lasersteuereinheit 10 den Laser 2 steuert. Die Lasersteuereinheit 10 kann Teil der Prozesssteuerungseinrichtung sein, sie kann jedoch auch in manchen Fällen Teil der Behandlungseinrichtung sein. Die Lasersteuereinheit 10 kann die Laserintensität des Lasers 2 sowie Umlenkspiegel 3 steuern, die den Strahlengang des Behandlungsstrahls 11 lenken und so die gezielte Behandlung einzelner Behandlungsflächenbereiche (20a, 20b, 20c) auf der Netzhaut 16 ermöglichen.

Zur besseren Steuerung des Lasers 2 ist eine Verarbeitungseinrichtung 19 als Teil der Prozesssteuerungseinrichtung vorgesehen, die eine präzise Verarbeitung von Aufnahmen/Kamerabildern der Kamera 6 ermöglicht und diese mit den bekannten und definierten Parametern der Beleuchtung der Netzhaut 16 verknüpft. Die Verarbeitungseinrichtung 19 kann unter Berücksichtigung von Aufnahmen der Netzhaut 16 Behandlungsparameter und Steuerungsdaten zur Steuerung der Behandlungseinrichtung einschließlich des Lasers 2 und der Umlenkspiegel 3 erzeugen. Die Verarbeitungseinrichtung ist Teil einer Prozesssteuerungseinrichtung, die auch zusätzliche Elemente in einem verteilten Rechnernetzwerk 101 umfassen kann. Dazu kann in dem Rechnernetzwerk 101 beispielsweise ein selbstlernendes System, ein Expertensystem und eine Datenbank vorgesehen sein. Die Verarbeitungseinrichtung 19 oder ein Element des verteilten Rechnernetzwerks kann auch bestimmen, ob und wann der Prozess angehalten und eine Aufforderung zur Freigabe der Fortsetzung des Prozesses und zur Durchführung der tatsächlichen Laserbehandlung ausgegeben wird. Diese Aufforderung kann direkt am Ort des Datenerfassungs- und Behandlungssystems ausgegeben werden, das in der Figur 7 dargestellt ist oder mittels einer Kommunikationsverbindung auch entfernt von dem Datenerfassungs- und Behandlungssystem. Die Verarbeitungseinrichtung 19 weist auch eine drahtlose Schnittstelle zu dem mobilen Endgerät 102 auf, das beispielsweise ein Smartphone sein kann.

Mittels eines Smartphones können beispielsweise vorläufige Tests oder Aufnahmen des Auges oder der Netzhaut durch einen potenziellen Patienten vor dem Besuch einer Behandlungseinrichtung oder eines Arztes durchgeführt werden. Die erfassten Daten können dann zur weiteren Analyse von dem Smartphone/Endgerät an die Verarbeitungseinrichtung 19 übermittelt werden. Sie können jedoch in vielen Fällen auch mittels einer Kommunikationsverbindung an das verteilte Rechnernetzwerk übermittelt werden. Die Verarbeitungseinrichtung 19 kann auch eine Bezahleinrichtung aufweisen, die eine Kommunikation mit dem Endgerät nutzt, um Zahlungen anzuweisen oder durch das Endgerät Zahlungen bestätigen zu lassen. Dies kann aus Gründen der Datensicherheit beispielsweise durch eine Near-field-Kommunikationsschnittstelle erfolgen.

Das Ophthalmoskop umfasst ferner einen Sensor 100, z.B. in Form einer Kamera, der die Messung der Pigmentierungsfarbe und Pigmentierungsintensität der Haut, der Haare und/oder der Iris des Patienten ermöglicht. Es kann auch eine Eingabevorrichtung vorgesehen sein, mit der solche gemessenen oder auch andere, den Patienten betreffende Parameter in das System eingegeben werden können. In jedem Fall werden diese Parameter an die Verarbeitungsvorrichtung 19 oder zu dem verteilten Rechnernetzwerk 101 gesendet, wo sie bei der Erzeugung der Steuerungsdaten, beispielsweise für die Bestrahlungsparameter, berücksichtigt werden.

Durch die Beleuchtung der Netzhaut mit bekannten Beleuchtungsparametern und deren Verknüpfung mit dem Bild der Netzhaut ist es möglich, ggf. Korrekturfaktoren für die Intensität der Laserbehandlung für jeden Behandlungsflächenbereichsbereich 20a, 20b, 20c auf der Netzhaut 16 durch die die Prozesssteuerungseinrichtung, beispielsweise konkret durch die Verarbeitungseinrichtung 19, den Behandlungsflächenbereichen bzw. Flächenabschnitten objektiviert zuzuordnen. Die Intensität des Laserstrahls ist dabei durch die Energie des Lasers, die Größe des Laserspots auf der Netzhaut und die Anzahl, Wiederholrate und Dauer des oder der Pulse sowie die Länge der Pausen zwischen den Pulsen (Tastverhältnis des Lasers) gegeben.

In vielen Bereichen der Augenheilkunde werden verschiedene Energiequellen, insbesondere Laser, zur Diagnose und Behandlung eingesetzt. In der Regel wird die gesamte eingestrahlte Energie vom biologischen Gewebe absorbiert und in Wärme umgewandelt, wobei die daraus resultierende Temperaturerhöhung den gewünschten Behandlungseffekt erzielt. So wird beispielsweise bei der Laser-Photokoagulation die Netzhaut des Auges gezielt thermisch koaguliert. Bei den herkömmlichen Bestrahlungen mit Bestrahlungszeiten um 100 ms entstehen Temperaturen von über 60° C. Auch bei der transpupillären Thermotherapie (TTT) werden Temperaturerhöhungen zum Erreichen eines Gefäßverschlusses genutzt. Bei der photodynamischen Therapie (PDT) wird ein zuvor injizierter Farbstoff durch Laserbestrahlung eines Therapielasers am Augenhintergrund aktiviert. Der Wirkstoff entfaltet seine Wirkung nur an den Zellen, an die er gebunden ist. Auch in diesem Fall wird fast die gesamte eingestrahlte Energie im Farbstoff und in der Netzhaut absorbiert und in Wärme umgewandelt. Während der jeweiligen Bestrahlungszeit (Pulsdauer) sollte bei relativ langer Behandlung und Strahlungspulsen in der Größenordnung von μs bis zu mehreren hundert Sekunden auf jeden Fall eine Temperaturerhöhung des behandelten biologischen Gewebes, insbesondere des Augenhintergrundes, vermieden werden, die zu einer unbeabsichtigten Schädigung von Bereichen der Netzhaut führt. Eine nicht-invasive Echtzeit-Temperaturbestimmung während einer ophthalmologischen Laserbehandlung ist seit langem gewünscht worden. Als Lösung wurde eine Technologie entwickelt, die im Rahmen der vorgestellten Behandlungseinrichtung eingesetzt werden kann und die bereits kurz mit Bezug auf Figur 2 beschrieben wurde und es erlaubt, die Gewebetemperatur während der thermischen Behandlung von biologischem Gewebe, insbesondere bei ophthalmologischen Behandlungen, mittels optoakustischer Verfahren zu überwachen. Im Einzelnen ist es bekannt, die durch eine Laserbehandlung erreichte Gewebetemperatur von biologischem Gewebe mit optoakustischen Techniken durch gepulste Laserbestrahlung zu bestimmen. Die Temperaturen werden am Augenhintergrund z.B. bei der selektiven Mikrophotokoagulation oder bei jeder anderen Behandlung von Erkrankungen der Netzhaut gemessen, bei der eine Strahlungsquelle wie z.B. ein Therapielaser eingesetzt wird. Ein spezieller Laserpuls, der zur Temperaturmessung vorgesehen ist, erzeugt eine thermische Gewebeausdehnung oder am Ende des Pulses eine thermische Gewebekontraktion, wobei sowohl die Ausdehnung als auch die Kontraktion Druckwellen erzeugen und wobei die Amplitude oder andere Charakteristika der Druckwellen zur Kalibrierung der Druckwellencharakteristika, z.B. der Amplitude, in Bezug auf die Temperatur oder auf eine komplexere Regelgröße mit einer definierten Beziehung zur Temperatur und zu einer Absorptionscharakteristik des Augenhintergrundes verwendet werden können. Aus der Änderung der nachfolgenden Druckwellencharakteristiken nach aufeinanderfolgenden Behandlungsimpulsen lassen sich anhand des Zusammenhangs zwischen der Temperatur des Gewebes und den Druckwellencharakteristiken, insbesondere der Druckwellenamplitude, die durch den Grüneisen-Koeffizienten definiert ist, die Temperaturerhöhung bzw. -abnahme und darüber hinaus die jeweiligen absoluten Temperaturen bestimmen. Es ist ein Aspekt der vorliegenden Erfindung, ein vereinfachtes Verfahren und eine Vorrichtung zur nicht-invasiven Bestimmung der oben genannten Größen oder der Temperatur an behandeltem biologischem Gewebe bereitzustellen, die auch eine Zielgröße bilden kann. Die gemessene Größe, beispielsweise die Temperatur, kann dann, wie oben bereits erläutert, zur Steuerung der Leistung des Therapielasers dienen. Die Zielgröße der Regelung kann beispielsweise die aktuelle Temperatur des behandelten Gewebes des Auges sein, die aus der Messung der Stärke der opto-akustischen Pulse oder anderer Parameter der detektierten Druckwellen ermittelt werden kann. So kann beispielsweise die Stärke der Druckwellen, ihre Amplitude, die durchschnittliche Amplitude oder die akkumulierte Energie gemessen oder bestimmt werden.

Die gemessene Stärke der Druckwellen ist neben der Intensität der Anregung abhängig von der Temperatur des Gewebes, in dem die Druckwellen erzeugt werden, der Absorption der zugehörigen Lasersignale auf dem Weg zum Gewebe und der Intensität der Absorption der Lasersignale im Gewebe (daher z.B.: die Pigmentierungsstärke). Im Bereich der Parameter, in dem die Behandlung im Allgemeinen stattfindet, wird die Stärke der detektierten Druckwellen dP durch die folgende Gleichung bestimmt:

$$dP = \mu\,\Gamma\,dH,$$

wobei dP die Variation der Stärke der Druckwellen, $\mu$ die Absorptionsstärke des Laserlichts im Gewebe, $\Gamma$ der Grüneisen-Koeffizient und dH die akkumulierte Laserstrahlenergie im Gewebe ist (die Wärmeableitung im Gewebe wird für kurze Zeitbereiche vernachlässigt). Da beide Parameter, die Gewebetemperatur und die Absorptionsstärke des Gewebes, in die gleiche Richtung weisen (hohe Gewebetemperatur und hohe Absorptionsstärke erfordern eine geringere Leistung des Therapielasers), ist die gemessene Stärke der Druckwellen eine geeignete Regelgröße zur Steuerung der Leistung des Therapielasers. Dabei ist es eine Möglichkeit, den Regelwert als solchen für die Steuerung des Therapielasers zu verwenden, eine andere Möglichkeit ist es aber auch, zunächst die aktuelle Temperatur des Gewebes zu bestimmen, z.B. anhand einer Kalibrierung, und dann den Therapielaser anhand des ermittelten Temperaturwertes zu steuern.

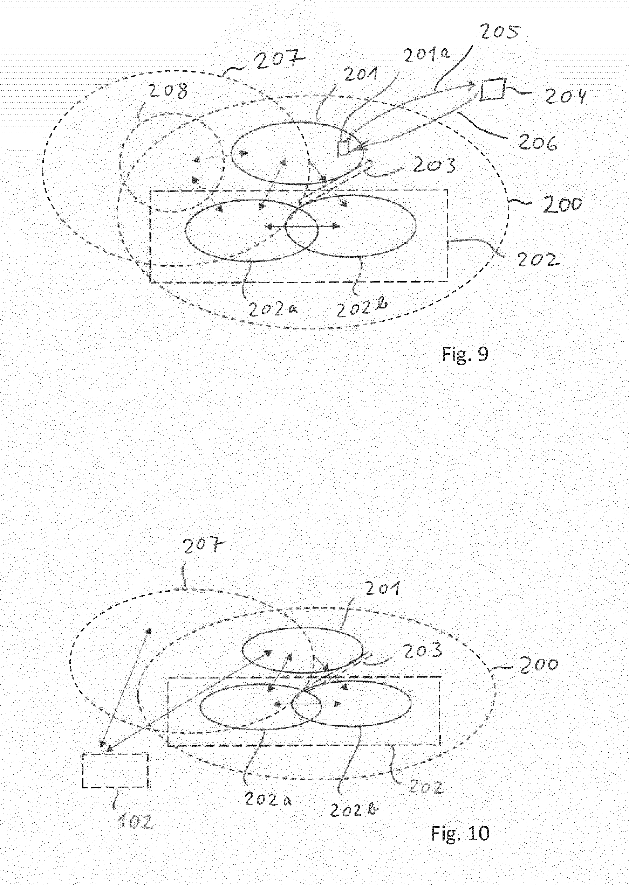

In der Figur 9 ist ein Prozesssystem 200 schematisch dargestellt, das ein Datenerfassungs- und Behandlungssystem 202 und eine Prozesssteuerungseinrichtung 201 umfasst. Das Datenerfassungssystem 202a des Datenerfassungs- und Behandlungssystems 202 weist ein Aufnahmesystem mit einer oder mehreren Kameras oder einem Netzhautscanner oder ähnlichen bildgebenden Einrichtungen auf, die die Erstellung einer Aufnahme, insbesondere einer optischen Aufnahme der Netzhaut oder beispielsweise einer tomographischen Aufnahme ermöglichen.

Die Datenerfassungseinrichtung weist ferner optional Sensoren zur Messung äußerer Merkmale des Patienten wie der Haar/Hautfarbe oder der Farbe der Iris auf, die unabhängig von der Netzhaut gemessen werden können. Zudem weist die Datenerfassungseinrichtung eine Dateneingabeeinrichtung zur manuellen Eingabe von Personen- und Gesundheitsdaten, beispielsweise in Form einer Tastatur sowie eine Kommunikationsschnittstelle zur Übernahme von Daten, beispielsweise von bereits existierenden Netzhautaufnahmen, die mit derselben oder eine er anderen Datenerfassungseinrichtung erfasst worden sind und gespeichert zur Verfügung stehen, oder von Krankenkassendaten, aus einer Datenbank oder von einer Verarbeitungseinrichtung auf. Die erfassten Daten werden an die Prozesssteuerungseinrichtung 201 übermittelt, die unter Berücksichtigung der Daten Steuerungsdaten für eine nachfolgende Behandlung ermittelt. Dabei kann die Prozesssteuerungseinrichtung zusätzlich Eingaben einer Bedienperson, beispielsweise eines Arztes, berücksichtigen. Solche Eingaben können beispielsweise Vorgaben für eine Behandlung oder Teile eines Behandlungsplans oder auch ein gesamter Behandlungsplan sein. Die Prozesssteuerungseinrichtung 201 kann am Ort des Datenerfassungs- und Behandlungssystems 202 vorgesehen sein oder entfernt davon, beispielsweise bei einer Realisierung in einer cloud. Innerhalb der Prozesssteuerungseinrichtung 201 ist ein Entscheidungsmodul 201a vorgesehen, das in Abhängigkeit von den von der Datenerfassungseinrichtung 202a erfassten Daten oder von den erzeugten Steuerungsdaten ein Unterbrechungssignal erzeugt, welches den automatisierten Gesamtprozess vor Beginn der Behandlung durch einen Unterbrechungsschritt 203 unterbrechen kann. Zudem wird ein Aufforderungssignal erzeugt und, wie durch den Pfeil 205 dargestellt, an eine Entscheidungsinstanz 204 übermittelt, die eine Fortsetzung des Gesamtprozesses freigeben kann, dargestellt durch den Pfeil 206. Alternativ kann auch ohne eine Kopplung an bestimmte Bedingungen unbedingt eine Unterbrechung mit einer Freigabeaufforderung erfolgen. Die Entscheidungsinstanz kann eine Person, beispielsweise ein Arzt, oder ein separates Datenverarbeitungssystem, beispielsweise mit einer selbstlernenden Einrichtung und/oder einem trainierten neuronalen Netz oder eine beide umfassende kooperative Einheit sein. Die Entscheidungsinstanz kann auch durch ein Entscheidungsmodul innerhalb des Prozesssystems gebildet sein. Die Freigabe kann auch Korrekturen und/oder Ergänzungen der Steuerungsdaten, insbesondere eines Behandlungsplans, mit umfassen.

Ist eine Freigabe 206 erfolgt, so wird mittels der Behandlungseinrichtung 202b eine Laserbehandlung durchgeführt. Die Steuerungsdaten dienen dann dazu, die Behandlungseinrichtung zu steuern. Die Steuerungsdaten können beispielsweise für verschiedene Behandlungsflächenbereiche oder Flächenabschnitte bestimmte Bestrahlungsintensitäten sowie eine Reihenfolge der Bestrahlung enthalten sowie bestimmte Gebiete der Netzhaut, die nicht bestrahlt werden sollen.

Die Prozesssteuereinrichtung oder Teile davon können baulich mit dem Datenerfassungs- und Behandlungssystem in einem Gerät zusammengefasst sein oder über eine Kommunikationsverbindung mit diesem verbunden sein.

In das Prozesssystem kann ein Bezahlsystem 207 oder ein Teil davon integriert sein. Beispielsweise kann das Prozesssystem ein Preisberechnungsmodul 208 enthalten, das in Abhängigkeit von den von der Datenerfassungseinrichtung 202a erfassten Daten oder von den erzeugten Steuerungsdaten einen Preis für eine Behandlung ermittelt und diesen beispielsweise an die Prozesssteuerungseinrichtung und/oder an das Datenerfassungs- und Behandlungssystem übermittelt. Die Prozesssteuerungseinrichtung kann den Preis beispielsweise an eine Bezahlstelle wie eine Krankenkasse zusammen mit einer Zahlaufforderung oder einer Aufforderung zur Freigabe der Behandlung übermitteln. Eine Übermittlung des Preises an das

Datenerfassungs- und Behandlungssystem kann ermöglichen, dass der zu behandelnde Patient den Preis wahrnehmen und bezahlen kann, beispielsweise mittels einer Bezahlschnittstelle, die in das Datenerfassungs- und Behandlungssystem integriert sein kann, zum Beispiel in Form einer üblichen NFC- Schnittstelle für Smartphones 102 oder einer Bezahleinrichtung für Bezahlkarten, wobei die Bezahlung dann durch eine Drahtlos- Kommunikation mit einem Bezahlinstitut, typischerweise einer Bank, in Gang gesetzt oder bestätigt werden kann.

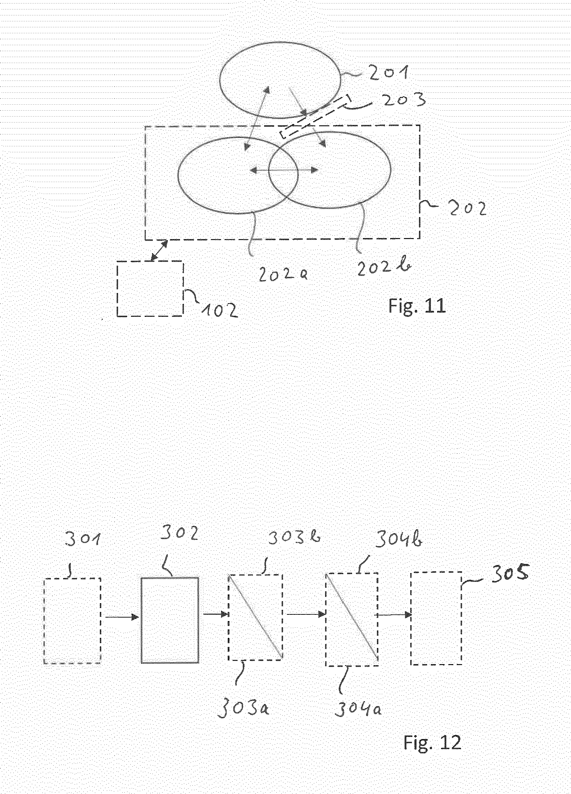

Eine unmittelbare Kommunikation eines mobilen Endgerätes 102 mit dem Datenerfassungs- und Behandlungssystem 202 ist in der Figur 11 gezeigt. Eine solche Kommunikation findet oft im Rahmen von Kommunikationsprotokollen und Drahtlosschnittstellen statt, die eine örtlich begrenzte Reichweite haben, beispielsweise auf Reichweiten unter 100 m. Beispiele hierfür sind WLAN/Wifi- Schnittstellen, Bluetooth und near-field-Kommunikation.

Mittels des Smartphones 102 können auch Personen- und Gesundheitsdaten, Identifikationszertifikate für ein Patientenkonto oder vorläufige Testergebnisse übermittelt werden, die mit dem Smartphone erfasst worden sind. Dies kann den gesamten Behandlungsprozess erheblich beschleunigen und auch dazu dienen, Fehler zu vermeiden, beispielsweise durch Fehlzuordnung von Daten.

In der Figur 10 ist eine Variante des Prozesssystems dargestellt, bei dem ein Smartphone 102 einerseits mit einem Bezahlsystem 207 und andererseits mit der Prozesssteuerungseinrichtung 201 mittels Kommunikationsverbindungen verbunden ist.

Beispielsweise kann nach Mittelung eines Behandlungspreises mittels des Smartphones eine Bestätigungsnachricht mit einer zertifizierten Identifikation des Patienten an eine Krankenkasse zur Initiierung einer Bezahlung geschickt werden. Diese Nachricht kann zunächst von dem Smartphone an die Prozesssteuerungseinrichtung übermittelt und von dieser an die bezahlende Krankenkasse übermittelt werden. Das Smartphone kann auch dazu benutzt werden, eine Zahlungsanweisung direkt an ein Bezahlinstitut, beispielsweise eine Bank des Patienten oder ein zertifiziertes Übermittlungsinstitut zu schicken, welches eine verschlüsselte Bezahlbestätigung entweder direkt an das Bezahlsystem 207 und/oder an das Smartphone schickt.

Eine weitere Funktion eines Smartphones kann in der Voraberfassung von Daten des Patienten bestehen. Das Smartphone kann ein Programm enthalten, das einen speziellen Sehtest oder eine Bildaufnahme des Auges und/oder der Netzhaut ermöglicht. Das Smartphone kann auch mit einem speziellen Aufnahmegerät koppelbar sein, das transportabel ist, über die notwendigen optischen Elemente verfügt und einem Patienten zur Erfassung von Netzhautaufnahmen zu Hause oder jedenfalls an einem Ort, der von dem Behandlungsort verschieden ist, zur Verfügung stehen kann. Das Smartphone kann dann dazu dienen, die Messdaten zu erfassen und an ein Datenerfassungs- und Behandlungssystem zu übermitteln. Es kann für eine Netzhautaufnahme auch beispielsweise eine Kamera des Smartphones genutzt werden. Das Aufnahmegerät kann dann über die notwendigen Elemente verfügen, um ein Bild der Netzhaut auf die Bildebene einer Smartphone-kamera zu projizieren.

Das Programm auf dem Smartphone kann auch die Erfassung von Personen - und Gesundheitsdaten ermöglichen, die mittels einer Schnittstelle an die Prozesssteuerungseinrichtung 201 übermittelt werden können. Diese Übermittlung kann durch eine Fern- Kommunikation, beispielsweise ein soziales Medium, mail oder SMS erfolgen oder in Anwesenheit des Patienten durch ein Nahbereichs- Kommunikationsmittel unmittelbar an die Prozesssteuerungseinrichtung 201 oder an das Datenerfassungs- und Behandlungssystem 202.

Der weitere Prozess der Datenerfassung durch die Datenerfassungseinrichtung 202a sowie die Erzeugung von Steuerungsdaten durch die Prozesssteuerungseinrichtung 201 kann in Abhängigkeit von den durch das Smartphone übermittelten, vorab erfassten Daten gestaltet werden.

In der Figur 12 ist schematisch ein Gesamtprozess dargestellt, der mittels des oben beschriebenen Prozesssystems durchgeführt oder organisiert werden kann.

In einem ersten, optionalen Verfahrensschritt 301 findet eine Vorab- Datenerfassung mittels eines mobilen Endgerätes statt. Die vorab erfassten Daten können an das Prozesssystem übermittelt werden. Sie können jedoch auch ausschließlich einer Voraborientierung des Patienten dienen. Die Daten können mittels einer Tastatur in das Endgerät eingegeben oder beispielsweise durch einen geeigneten Sehtest mittels des Endgerätes erfasst werden.

In einem zweiten Prozessschritt 302 wird mit der stationären Datenerfassungseinrichtung eine Erfassung von Daten durchgeführt, wobei eine bildgebende Aufnahme der Netzhaut im Mittelpunkt steht. Zusätzlich können in diesem Schritt weitere Daten des Patienten erfasst, gemessen oder eingegeben werden. Auch Daten über bereits erfolgte Untersuchungen und/oder Behandlungen können dabei ermittelt und übernommen werden.

Im folgenden Prozessschritt 303a werden erfasste Daten an die Prozesssteuerungseinrichtung 201 übermittelt und dort werden Steuerungsdaten erzeugt. Darauf folgend kann in einem folgenden Schritt 303b durch die Prozesssteuerungseinrichtung der Prozess unterbrochen werden und es kann eine Freigabeaufforderung erzeugt und ausgegeben/übermittelt werden.

Erfolgt durch ein geeignetes Signal eine Freigabe, so wird in einem nächsten, potenziellen Schritt 304a durch ein Preisberechnungsmodul 208 der Prozesssteuerungseinrichtung ein Preis berechnet oder bestimmt und eine Zahlungsaufforderung oder eine Anforderung der Kostenübernahme erzeugt und ausgegeben.

Im nächsten Schritt 304b kann durch die Prozesssteuerungseinrichtung ein Zahlungseingang nachgewiesen oder bestätigt werden. Erfolgt dies, so kann in einem nachfolgenden Schritt 305 die tatsächliche Laserbehandlung erfolgen.

Für eine Nachsorge können verschiedene Schritte vorgesehen werden, bei denen wieder das Endgerät, das beispielsweise ein Smartphone sein kann, unterstützend eingesetzt werden kann, wie beispielsweise weitere Tests/Sehtests oder Bildaufnahmen, eine Patientenbefragung und ähnliche Aktivitäten.

Das vorgestellte Prozesssystem ermöglicht eine Behandlung einer Vielzahl von Patienten mit einem reduzierten Aufwand, wodurch Zeit und Kosten gespart werden können und wodurch beispielsweise auch eine größere Zahl von Patienten von den Vorteilen einer prophylaktischen Behandlung profitieren kann.

## Patentansprüche

1. Prozesssystem (200) zur Behandlung der Netzhaut (16) eines Auges (4) mittels einer Behandlungslichtquelle (2), insbesondere eines Behandlungslasers,

   - mit einem Datenerfassungs-und Behandlungssystem (202), das eine Datenerfassungseinrichtung (202a) zur Erfassung wenigstens einer Aufnahme der Netzhaut, insbesondere durch eine mit einem Sensor ausgestattete Aufnahmeeinrichtung oder durch Übernahme von bereits existierenden Messdaten, und eine Behandlungseinrichtung (202b) zur Behandlung der Netzhaut mit der Behandlungslichtquelle in Abhängigkeit von der wenigstens einen Aufnahme der Netzhaut umfasst, und
   - mit einer Prozesssteuerungseinrichtung (201), die mit dem Datenerfassungs-und Behandlungssystem, insbesondere mit der Datenerfassungseinrichtung einerseits und mit der Behandlungseinrichtung andererseits verbunden ist und die dazu eingerichtet ist, in Abhängigkeit von den durch die Datenerfassungseinrichtung erfassten Daten Steuerungsdaten zur Steuerung der Behandlungseinrichtung zu erzeugen, **dadurch gekennzeichnet, dass** das Prozesssystem dazu eingerichtet ist, die Datenerfassung, die Erzeugung von Steuerungsdaten sowie die Behandlung automatisiert ablaufen zu lassen, wobei das Prozesssystem entweder dazu eingerichtet ist, vor Beginn der Behandlung mittels der Behandlungseinrichtung den weiteren Verlauf des Verfahrens bis zu einer Freigabe (206) zu unterbrechen und nach einer Freigabe fortzusetzen, oder dazu eingerichtet ist, dass durch die Prozesssteuerungseinrichtung entschieden wird, ob das Verfahren einschließlich der Behandlung automatisiert ohne eine Unterbrechung durchgeführt oder vor Beginn der Behandlung unterbrochen und nur nach einer Freigabe automatisiert fortgesetzt wird.

2. Prozesssystem nach Anspruch 1, **dadurch gekennzeichnet, dass** die Prozesssteuerungseinrichtung (201) wenigstens zum Teil durch eine von dem Datenerfassungs-und Behandlungssystem (202) gesondert vorgesehene und mit diesem über Kommunikationsverbindungen verbundene Datenverarbeitungseinrichtung gebildet ist, wobei diese Datenverarbeitungseinrichtung insbesondere wenigstens teilweise in Form eines Netzwerks (101) mit mehreren Recheneinrichtungen realisiert ist.

3. Prozesssystem nach Anspruch 1 oder 2, **dadurch gekennzeichnet, dass** die Datenerfassungseinrichtung einerseits und die Behandlungseinrichtung andererseits als Teile des Datenerfassungs- und Behandlungssystems baulich in einem einzigen Gerät integriert sind.

4. Prozesssystem nach Anspruch 1, 2 oder 3, **dadurch gekennzeichnet, dass** es ein Bezahlsystem (207, 208) oder wenigstens Elemente eines Bezahlsystems enthält.

5. Prozesssystem nach Anspruch 4, **gekennzeichnet durch** ein in das Prozesssystem integriertes Preisberechnungsmodul (208), das dazu eingerichtet ist, unter Berücksichtigung der durch die Datenerfassungseinrichtung (202a) erfassten Daten und/oder unter Berücksichtigung der durch die Prozesssteuerungseinrichtung (201) ermittelten Steuerungsdaten einen Preis für eine Behandlung zu ermitteln und eine Zahlungsaufforderung auszugeben, wobei das Prozesssystem insbesondere weiter dazu eingerichtet ist, die Erzeugung oder Übermittlung von Steuerungsdaten und/oder die Behandlung zu verzögern, bis ein Bezahlvorgang validiert worden ist.

6. Prozesssystem nach Anspruch 4 oder 5, **gekennzeichnet durch** ein Abrechnungsmodul (208), das dazu eingerichtet ist, Bezahldaten von dem Benutzer und/oder von einem Bezahlinstitut zu empfangen, die Bezahldaten zu validieren und nach erfolgreicher Validierung die Behandlung freizugeben oder zu starten.

7. Prozesssystem nach einem der Ansprüche 1, bis 6, **dadurch gekennzeichnet, dass** es dazu eingerichtet ist, mit einem mobilen Endgerät (102) zusammenzuwirken und von diesem wenigstens Daten einer der folgenden Kategorien zu empfangen und zu verarbeiten:

Patientenidentifikationsdaten
Messdaten von Messungen, die mittels des Endgerätes durchgeführt werden,
Bezahldaten.

8. Prozesssystem nach Anspruch 7, **dadurch gekennzeichnet, dass** es dazu eingerichtet ist, die Datenerfassungseinrichtung (202a) zur Erfassung wenigstens einer Aufnahme der Netzhaut (16) in Abhängigkeit von durch das mobile Endgerät (102) an das Prozesssystem (200) übermittelten Patientenidentifikationsdaten und/oder von Messdaten von Messungen zu steuern, die mittels des Endgerätes durchgeführt wurden.

9. Prozesssystem nach Anspruch 7 oder 8, **dadurch gekennzeichnet, dass** die Prozesssteuerungseinrichtung (201) die Steuerungsdaten zur Steuerung der Behandlungseinrichtung (202b) in Abhängigkeit von durch das mobile Endgerät (102) an das Prozesssystem übermittelten Patientenidentifikationsdaten und/oder von Messdaten von Messungen erzeugt, die mittels des Endgerätes (102) durchgeführt wurden.

10. Prozesssystem nach einem der Ansprüche 1 bis 9, **dadurch gekennzeichnet, dass** ein mögliches und durch die Prozesssteuerungseinrichtung (201) auswählbares Behandlungsverfahren eine Behandlung der Netzhaut (16) mit einer Bestrahlungsintensität vorsieht, die im Gewebe der Netzhaut Temperaturen von weniger als 58 Grad Celsius, insbesondere mit einer Höchsttemperatur zwischen 48 Grad und 58 Grad, weiter insbesondere mit einer Höchsttemperatur zwischen 50 Grad und 55 Grad erzeugt.

11. Datenerfassungs-und Behandlungssystem zur Behandlung der Netzhaut eines Auges mittels einer Behandlungslichtquelle (2), insbesondere eines Behandlungslasers, mit
einer Datenerfassungseinrichtung (202a), die wenigstens eine Aufnahme der Netzhaut erfasst und mit einer Behandlungseinrichtung (202b) zur Behandlung der Netzhaut mit der Behandlungslichtquelle in Abhängigkeit von der wenigstens einen Aufnahme der Netzhaut, wobei die Datenerfassungseinrichtung dazu eingerichtet ist, erste Daten mittels einer Kommunikationsverbindung an eine Prozesssteuerungseinrichtung (201) zu senden und wobei die Behandlungseinrichtung dazu eingerichtet ist, zweite Daten mittels einer Kommunikationsverbindung von der Prozesssteuerungseinrichtung zu empfangen, **dadurch gekennzeichnet, dass** das Datenerfassungs- und Behandlungssystem (202) dazu eingerichtet ist, mit einem mobilen Endgerät, insbesondere einem Mobiltelefon über eine Funkschnittstelle Daten auszutauschen, die eine Reichweite von weniger als 30 m, insbesondere weniger als 15 m hat, insbesondere eine Bluetooth-, WLAN- oder NFC-Schnittstelle und/oder dass das Datenerfassungs- und Behandlungssystem (202) eine Bezahlschnittstelle aufweist.

12. Verfahren zur Behandlung der Netzhaut (16) eines Auges mit einem Prozesssystem (200) mit einem Datenerfassungs-und Behandlungssystem (202), das eine Datenerfassungseinrichtung (202a) und eine Behandlungseinrichtung (202b) zur Behandlung der Netzhaut mit einer Behandlungslichtquelle (2) in Abhängigkeit von der wenigstens einen Aufnahme der Netzhaut sowie eine Prozesssteuerungseinrichtung 201) umfasst, wobei die Datenerfassungseinrichtung wenigstens eine Aufnahme der Netzhaut erfasst und an die Prozesssteuerungseinrichtung übermittelt und die Prozesssteuerungseinrichtung in Abhängigkeit von den durch die Datenerfassungseinrichtung erfassten Daten selbstständig Steuerungsdaten zur Steuerung einer Behandlungseinrichtung erzeugt,

**dadurch gekennzeichnet, dass** das Verfahren vor Beginn der Behandlung durch die Behandlungseinrichtung unterbrochen und nur nach einer Freigabe selbsttätig fortgesetzt wird.

Fig.1

Fig.2

$d_4$

$d_1$ $d_2$ $d \rightarrow$ Fig 3

$d_3$

21

C

b

a

20b

20a

Fig 4

20a

s2

s1

n2 n1 C t1 t2

i1

i2

20c

20b

Fig. 5

f6

s2

f7

n2 C t2

f8

f1

i2

20a 20b

f

e

f5

f4

20c

f3

f2 Fig 6

Fis.7

Fig 8

Fig. 9

Fig. 10

201

203

202

202a      202b

102

Fig. 11

301      302      303b      304b

305

303a      304a

Fig. 12

Europäisches
Patentamt

European
Patent Office

Office européen
des brevets

# EUROPÄISCHER RECHERCHENBERICHT

**Nummer der Anmeldung**

EP 23 21 9548

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | US 2003/032949 A1 (SCHUELE GEORGE [DE] ET AL) 13. Februar 2003 (2003-02-13) <br> * Anspruch 9 * <br> * Absätze [0020], [0028], [0033] * <br> ----- | 1-10,12 | INV. <br> A61F9/008 |
| X <br><br> Y | WO 2023/197057 A1 (PULSEMEDICA CORP [CA]) 19. Oktober 2023 (2023-10-19) <br> * Absätze [0050], [0051], [0063], [0065], [0066], [0101] - [0103], [0113], [0115], [0117] * <br> ----- | 1-4,7-9, 12 <br><br> 5,6 | |
| X | US 2021/290437 A1 (AKIYAMA HIROSHI [JP]) 23. September 2021 (2021-09-23) <br> * Abbildungen 1,3 * <br> * Absätze [0001], [0050], [0053], [93ff], [0140], [0142] - [0144] * <br> ----- | 1-3,12 | |
| X | US 9 931 171 B1 (PEYMAN GHOLAM A [US]) 3. April 2018 (2018-04-03) <br> * Abbildungen 2,5 * <br> * Spalte 3, Zeile 28 - Zeile 43 * <br> * Spalte 16, Zeile 33 - Zeile 59 * <br> * Spalte 17, Zeile 10 - Zeile 13 * <br> * Spalte 19, Zeile 30 - Spalte 20, Zeile 59 * <br> * Spalte 37, Zeile 28 - Zeile 40 * <br> ----- | 1,2, 7-10,12 | |
| Y | US 2006/290885 A1 (COVANNON EDWARD [US] ET AL) 28. Dezember 2006 (2006-12-28) <br> * Absätze [0023], [0027], [0029] * <br> ----- | 5,6 | RECHERCHIERTE SACHGEBIETE (IPC) <br><br> A61F |
| X | US 2014/330352 A1 (LUTTRULL JEFFREY K [US] ET AL) 6. November 2014 (2014-11-06) <br> * Absätze [0019], [0025], [0027], [0028], [0032], [0033] * <br> * Anspruch 1 * <br> ----- | 11 | |

-/--

Der vorliegende Recherchenbericht wurde für alle Patentansprüche erstellt

| Recherchenort | Abschlußdatum der Recherche | Prüfer |
|---|---|---|
| München | 20. Juni 2024 | Jansen, Birte |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

................................................................
& : Mitglied der gleichen Patentfamilie, übereinstimmendes Dokument

EPO FORM 1503 03.82 (P04C03)

Europäisches
Patentamt

European
Patent Office

Office européen
des brevets

# EUROPÄISCHER RECHERCHENBERICHT

**Nummer der Anmeldung**

EP 23 21 9548

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2013/116670 A1 (ARTSYUKHOVICH ALEXANDER [US] ET AL) 9. Mai 2013 (2013-05-09) * Abbildung 1 * * Absätze [0001], [0019], [0022], [0024], [0077] * ----- | 11 | |
| X | US 2003/208189 A1 (PAYMAN GHOLAM A [US]) 6. November 2003 (2003-11-06) * Abbildung 26 * * Absätze [0001], [0078], [0083] * ----- | 11 | |

**RECHERCHIERTE SACHGEBIETE (IPC)**

Der vorliegende Recherchenbericht wurde für alle Patentansprüche erstellt

| Recherchenort | Abschlußdatum der Recherche | Prüfer |
|---|---|---|
| München | 20. Juni 2024 | Jansen, Birte |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

..............................................................................

& : Mitglied der gleichen Patentfamilie, übereinstimmendes Dokument

EPO FORM 1503 03.82 (P04C03)

Seite 2 von 2

Europäisches
Patentamt

European
Patent Office

Office européen
des brevets

**Nummer der Anmeldung**

EP 23 21 9548

## GEBÜHRENPFLICHTIGE PATENTANSPRÜCHE

Die vorliegende europäische Patentanmeldung enthielt bei ihrer Einreichung Patentansprüche, für die eine Zahlung fällig war.

☐ Nur ein Teil der Anspruchsgebühren wurde innerhalb der vorgeschriebenen Frist entrichtet. Der vorliegende europäische Recherchenbericht wurde für jene Patentansprüche erstellt, für die keine Zahlung fällig war, sowie für die Patentansprüche, für die Anspruchsgebühren entrichtet wurden, nämlich Patentansprüche:

☐ Keine der Anspruchsgebühren wurde innerhalb der vorgeschriebenen Frist entrichtet. Der vorliegende europäische Recherchenbericht wurde für die Patentansprüche erstellt, für die keine Zahlung fällig war.

## MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

    Siehe Ergänzungsblatt B

☒ Alle weiteren Recherchengebühren wurden innerhalb der gesetzten Frist entrichtet. Der vorliegende europäische Recherchenbericht wurde für alle Patentansprüche erstellt.

☐ Da für alle recherchierbaren Ansprüche die Recherche ohne einen Arbeitsaufwand durchgeführt werden konnte, der eine zusätzliche Recherchengebühr gerechtfertigt hätte, hat die Recherchenabteilung nicht zur Zahlung einer solchen Gebühr aufgefordert.

☐ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind, nämlich Patentansprüche:

☐ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen, nämlich Patentansprüche:

☐ Der vorliegende ergänzende europäische Recherchenbericht wurde für die Teile der Anmeldung erstellt, die sich auf die zuerst in den Patentansprüchen erwähnte Erfindung beziehen (Regel 164 (1) EPÜ).

Europäisches
Patentamt

European
Patent Office

Office européen
des brevets

**MANGELNDE EINHEITLICHKEIT
DER ERFINDUNG
ERGÄNZUNGSBLATT B**

**Nummer der Anmeldung**

EP 23 21 9548

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

1. Ansprüche: 1-10, 12

   Augenlasersystem mit Prozessunterbrechung
   - - -

2. Anspruch: 11

   Augenlasersystem mit Steuerung über mobiles Endgerät
   - - -

**ANHANG ZUM EUROPÄISCHEN RECHERCHENBERICHT
ÜBER DIE EUROPÄISCHE PATENTANMELDUNG NR.**

EP 23 21 9548

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

20-06-2024

| Im Recherchenbericht angeführtes Patentdokument | Datum der Veröffentlichung | Mitglied(er) der Patentfamilie | Datum der Veröffentlichung |
|---|---|---|---|
| US 2003032949 A1 | 13-02-2003 | DE 10135944 A1 | 20-02-2003 |
| | | EP 1279385 A1 | 29-01-2003 |
| | | US 2003032949 A1 | 13-02-2003 |
| WO 2023197057 A1 | 19-10-2023 | KEINE | |
| US 2021290437 A1 | 23-09-2021 | JP 7164338 B2 | 01-11-2022 |
| | | JP 2020006000 A | 16-01-2020 |
| | | US 2021290437 A1 | 23-09-2021 |
| | | WO 2020012840 A1 | 16-01-2020 |
| US 9931171 B1 | 03-04-2018 | KEINE | |
| US 2006290885 A1 | 28-12-2006 | KEINE | |
| US 2014330352 A1 | 06-11-2014 | KEINE | |
| US 2013116670 A1 | 09-05-2013 | AU 2012336362 A1 | 16-01-2014 |
| | | AU 2015210430 A1 | 03-09-2015 |
| | | CA 2842025 A1 | 16-05-2013 |
| | | CN 103747757 A | 23-04-2014 |
| | | CN 109620136 A | 16-04-2019 |
| | | EP 2731534 A1 | 21-05-2014 |
| | | ES 2604713 T3 | 08-03-2017 |
| | | JP 6050369 B2 | 21-12-2016 |
| | | JP 2014532514 A | 08-12-2014 |
| | | US 2013116670 A1 | 09-05-2013 |
| | | WO 2013070300 A1 | 16-05-2013 |
| US 2003208189 A1 | 06-11-2003 | AU 2003261141 A1 | 02-02-2004 |
| | | US 2003208189 A1 | 06-11-2003 |
| | | WO 2004006794 A1 | 22-01-2004 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1875857 A1

- EP 1279385 A1